(19) Europäisches Patentamt / European Patent Office / Office européen des brevets

(11) **EP 3 577 237 B1**

(12) **EUROPEAN PATENT SPECIFICATION**

(45) Date of publication and mention of the grant of the patent:
**19.07.2023 Bulletin 2023/29**

(21) Application number: **18703944.1**

(22) Date of filing: **01.02.2018**

(51) International Patent Classification (IPC):
**C12Q 1/6886** (2018.01)

(52) Cooperative Patent Classification (CPC):
**C12Q 1/6886; C12Q 2600/156**

(86) International application number:
**PCT/EP2018/052473**

(87) International publication number:
**WO 2018/141828 (09.08.2018 Gazette 2018/32)**

(54) **METHOD FOR INDICATING A PRESENCE OR NON-PRESENCE OF PROSTATE CANCER IN INDIVIDUALS WITH PARTICULAR CHARACTERISTICS**

VERFAHREN ZUR ANZEIGE DER ANWESENHEIT ODER ABWESENHEIT VON PROSTATAKREBS BEI PERSONEN MIT BESONDEREN EIGENSCHAFTEN

PROCÉDÉ D'INDICATION DE LA PRÉSENCE OU DE LA NON-PRÉSENCE D'UN CANCER DE LA PROSTATE CHEZ DES INDIVIDUS PRÉSENTANT DES CARACTÉRISTIQUES PARTICULIÈRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.02.2017 SE 1750080**

(43) Date of publication of application:
**11.12.2019 Bulletin 2019/50**

(73) Proprietor: **Phadia AB**
**751 37 Uppsala (SE)**

(72) Inventor: **GRÖNBERG, Henrik**
**112 18 Stockholm (SE)**

(74) Representative: **Brann AB**
**P.O. Box 3690**
**Sveavägen 63**
**103 59 Stockholm (SE)**

(56) References cited:
WO-A1-2012/029080    WO-A1-2014/079865
WO-A2-2012/031207    WO-A2-2013/172779

- **MARKUS ALY ET AL: "Polygenic Risk Score Improves Prostate Cancer Risk Prediction: Results from the Stockholm-1 Cohort Study", EUROPEAN UROLOGY, ELSEVIER, AMSTERDAM, NL, vol. 60, no. 1, 8 January 2011 (2011-01-08), pages 21-28, XP028223801, ISSN: 0302-2838, DOI: 10.1016/J.EURURO.2011.01.017 [retrieved on 2011-01-11]**
- **J. GUDMUNDSSON ET AL: "Genetic Correction of PSA Values Using Sequence Variants Associated with PSA Levels", SCIENCE TRANSLATIONAL MEDICINE, vol. 2, no. 62, 15 December 2010 (2010-12-15), pages 62ra92-62ra92, XP055073558, ISSN: 1946-6234, DOI: 10.1126/scitranslmed.3001513**

**Description**

FIELD OF THE INVENTION

The present invention relates generally to the detection and identification of various forms of genetic markers, and various forms of proteins, which have the potential utility as diagnostic markers. In particular, the present invention relates to the simultaneous use of multiple diagnostic markers for improved detection of prostate cancer and in particular aggressive forms of prostate cancer. More particularly, the present invention relates to the simultaneous use of multiple diagnostic markers for improved detection of prostate cancer for men that have particular genetic characteristics.

BACKGROUND OF THE INVENTION

The measurement of serum prostate specific antigen (PSA) is widely used for the screening and early detection of prostate cancer (PCa). As discussed in the public report "Polygenic Risk Score Improves Prostate Cancer Risk Prediction: Results from the Stockholm-1 Cohort Study" by Markus Aly and co-authors as published in EUROPEAN UROLOGY 60 (2011) 21-28, serum PSA that is measurable by current clinical immunoassays exists primarily as either the free "non-complexed" form (free PSA), or as a complex with a-lantichymotrypsin (ACT). The ratio of free to total PSA in serum has been demonstrated to significantly improve the detection of PCa. Other factors, like age and documented family history may also improve the detection of PCa further. The measurement of genetic markers related to PCa, in particular single nucleotide polymorphisms (SNP), is an emerging modality for the screening and early detection of prostate cancer. Analysis of multiple PCa related SNPs can, in combination with biomarkers like PSA and with general information about the patient improve the risk assessment through a combination of several SNPs into a genetic score.

The screening and early detection of prostate cancer is a complicated task, and to date no single biomarker has been proven sufficiently good for specific and sensitive mapping of the male population. Therefore, attempts have been spent on combining biomarker levels in order to produce a formula which performs better in the screening and early detection of PCa. The most common example is the regular PSA test, which in fact is an assessment of "free" PSA and "total" PSA. PSA exists as one "non-complex" form and one form where PSA is in complex formation with alpha-lantichymotrypsin. Another such example is the use of combinations of concentrations of free PSA, total PSA, and one or more pro-enzyme forms of PSA for the purpose of diagnosis, as described in WO03100079 (METHOD OF ANALYZING PROENZYME FORMS OF PROSTATE SPECIFIC ANTIGEN IN SERUM TO IMPROVE PROSTATE CANCER DETECTION). The one possible combination of PSA concentrations and pro-enzyme concentrations that may result in improved performance for the screening and early detection of PCa is the phi index. Phi was developed as a combination of PSA, free PSA, and a PSA precursor form [-2]proPSA to better detecting PCa for men with a borderline PSA test (e.g. PSA 2-10ng/mL) and non-suspicious digital rectal examination, as disclosed in the report "Cost-effectiveness of Prostate Health Index for prostate cancer detection" by Nichol MB and co-authors as published in BJU Int. 2011 Nov 11. doi: 10.1111/j.1464-410X.2011.10751.x.. Another such example is the combination of psp94 and PSA, as described in US2012021925 (DIAGNOSTIC ASSAYS FOR PROSTATE CANCER USING PSP94 AND PSA BIOMARKERS).

There are other biomarkers of potential diagnostic or prognostic value for assessing if a patient suffers from PCa, including MIC-1 as described in the report "Macrophage Inhibitory Cytokine 1: A New Prognostic Marker in Prostate Cancer" by David A. Brown and co-authors as published in Clin Cancer Res 2009;15(21):OF1-7.

Attempts to combine information from multiple sources into one algorithmic model for the prediction of PCa risk has been disclosed in the past. In the public report "Blood Biomarker Levels to Aid Discovery of Cancer-Related Single-Nucleotide Polymorphisms: Kallikreins and Prostate Cancer" by Robert Kleins and co-authors as published in Cancer Prev Res (2010), 3(5):611-619, the authors discuss how blood biomarkers can aid the discovery of novel SNP, but also suggest that there is a potential role for incorporating both genotype and biomarker levels in predictive models.

Furthermore, this report provides evidence that the non-additive combination of genetic markers and biomarkers in concert may have predictive value for the estimation of PCa risk. Later, Xu and co-inventors disclosed a method for correlating genetic markers with high grade prostate cancer, primarily for the purpose of identifying subjects suitable for chemopreventive therapy using 5-alpha reductase inhibitor medication (e.g. dutasteride or finasteride) in the patent application WO2012031207. In addition, WO2013172779 and WO2014079865 describe the feed of multiple sources of information into an algorithm that estimates the risk for PCa for a complete population. In concert, these public disclosures summarizes the prior art of combining genetic information and biomarker concentration for the purpose of estimating PCa risk, also for high grade cancers.

The current performance of the PSA screening and early detection is approximately a sensitivity of 80% and specificity of 30%. It is estimated that approximately 65% will undergo unnecessary prostate biopsy and that 15-20% of the clinically relevant prostate cancers are missed in the current screening. In the United States alone, about 1 million biopsies are performed every year, which results in about 192 000 new cases being diagnosed. Hence, also a small improvement of diagnostic performance will result both in major savings in healthcare expenses due to fewer biopsies and in less human suffering from invasive diagnostic procedures.

The current clinical practice (in Sweden) is to use total PSA as biomarker for detection of asymptomatic and early prostate cancer. The general cutoff value for further evaluation with a prostate biopsy is 3 ng/mL. However, due to the negative consequences of PSA screening there is no organized PSA screening recommended in Europe or North America today.

It is particularly important to accurately identify aggressive prostate cancer (aPCa) in individuals because the sooner an individual is provided treatment, the greater likelihood of the cancer being cured. The identification of aPCa is however difficult, partly because larger cohorts are required to provide a sufficient number of cases and controls in the development of statistical models. Hence, the availability of predictive models for aPCa is low. It is even more difficult to design models for subgroups in a population, because the required cohort size to at all be capable of designing such a model is very large.

Accordingly, there is a need in the art to identify improved models for predicting prostate cancer or aggressive prostate cancer.

## SUMMARY OF THE INVENTION

The present invention is based on the discovery that the combination of diagnostic markers of different origin may improve the ability to detect PCa (Prostate Cancer) or aPCa (aggressive Prostate Cancer) in a particular subpopulation of men with particular genetic characteristics. This finding can result in major savings for the society, because cancers and in particular aggressive cancers that are identified early are more easily treatable.

Accordingly, one aspect of the present invention provides a method for indicating a presence or non-presence of a prostate cancer (PCa) in an individual, said method comprising the steps of:

a) performing a genetic analysis of a biological sample obtained from said individual, comprising determining a presence or non-presence of risk allele(s) of a group of Single Nucleotide Polymorphism (SNP) related to a PCa Genetic Subpopulation (PCaGS), wherein if at least one of said risk alleles is/are present in said sample, said individual is determined to belong to said PCaGS, and if said at least one risk alleles of said group of SNPs is not present in said sample, said individual is determined not to belong to said PCaGS, wherein said group of SNPs consists of rs16901979, rs7818556, rs12793759 and rs138213197;

b) if in step a) said individual is determined to belong to a PCaGS, then determine and characterize one or more additional PCa related parameter(s) in said individual to indicate a presence or a non-presence of PCa in said PCaGS individual, wherein said one or more additional PCa related parameter(s) in said individual comprises measuring a total PSA value in said individual;

c) if said individual in step a) is determined not to belong to a PCaGS, then

i) determine a presence or concentration of a defined amount of PCa related biomarker(s) in said individual;

ii) determine a PCa related genetic status by determining a presence or absence of a defined amount of one or more risk alleles of a SNP(s) related to PCa in said individual;

iii) combine data from said individual regarding said presence or concentration of a defined amount of PCa related biomarker(s), and data from said individual regarding a PCa related genetic status to form a general PCa population composite value;

iv) correlate said general PCa population composite value to the presence or non-presence of PCa in said individual by comparing the general PCa population composite value to a pre-determined cut-off value established with control samples of known general population PCa and control samples of non-presence of PCa, wherein said defined amount of one or more risk alleles of an SNP related to PCa comprises SNPs present in any one of the following lists:

rs138213197, rs7818556, rs6983267, rs10993994, rs12793759, rs16901979, rs9911515, rs1016343, rs7106762, rs6579002, rs16860513, rs5945619, rs16902094, rs10896437, rs651164, rs7679673, rs13265330, rs2047408, rs10107982, rs620861, rs9297746, rs1992833, rs7213769, rs2710647, rs888507, rs17021918, rs12500426, rs2028900, rs7102758, rs16901922, rs6062509, rs2659051, rs12543663, rs4699312, rs11091768, rs3120137, rs6794467, rs10086908, rs2315654, rs12151618, rs747745, rs1009, rs2132276, rs2735839, rs11568818, rs684232, rs9364554, rs2660753, rs10807843, rs1933488, rs17467139, rs12947919, rs2331780, rs1894292, rs2107131, rs6545962, rs11649743, rs758643, rs2297434, rs902774, rs17224342, rs5918762, rs17138478, rs3019779, rs1873555, rs12946864, rs12475433, rs3765065, rs4871779, rs10875943, rs11601037, rs6489721, rs11168936, rs9297756, rs11900952, rs6569371, rs7752029, rs5934705, rs3745233, rs1482679, rs749264, rs6625760, rs5978944, rs2366711, rs5935063, rs10199796, rs2473057, rs4925094, rs3096702, rs12490248, rs4245739, rs10094059, rs306801, rs2823118, rs2025645, rs9359428, rs10178804, rs6090461, rs2270785 , rs16901841, and rs2465796 (list 1);

and/or

rs582598, rs439378, rs2207790, rs1046011, rs10458360, rs7525167, rs10489871, rs7529518, rs4245739, rs4512641, rs10178804, rs11900952, rs1873555, rs10191478, rs6755901, rs6545962, rs721048, rs2710647, rs12612891, rs2028900, rs1009, rs12233245, rs6760417, rs10496470, rs10199796, rs12475433, rs16860513, rs12151618, rs3765065, rs13017302, rs12988652, rs871688, rs749264, rs3771570, rs4346531, rs6770955, rs12637074, rs2660753, rs13319878, rs6437715, rs2162185, rs1515542, rs2270785, rs9830294, rs1439024, rs6762443, rs888507, rs6794467, rs12490248, rs1477886, rs4833103, rs3796547, rs17779822, rs2366711, rs16849146, rs1894292, rs12640320, rs3805284, rs12500426, rs4699312, rs17021918, rs7679673, rs2047408, rs2647262, rs12506850, rs7658048, rs2078277, rs12505546, rs13113975, rs4246742, rs2736098, rs401681, rs11134144, rs10060513, rs40485, rs2087724, rs1482679, rs16901841, rs1295683, rs2070874, rs7752029, rs2018334, rs9358913, rs1140809, rs409558, rs3096702, rs9267911, rs2025645, rs9359428, rs6569371, rs2813532, rs1933488, rs712242, rs6934898, rs9456490, rs651164, rs3120137, rs9364554, rs9457937, rs10486562, rs10807843, rs7801918, rs6962297, rs2465796, rs6957416, rs7777631, rs2272316, rs6961773, rs2132276, rs13265330, rs16887736, rs2911756, rs2272668, rs2339654, rs1380862, rs9297746, rs12543663, rs10086908, rs16901922, rs1016343, rs17832285, rs16901979, rs4871779, rs10107982, rs16902094, rs620861, rs17467139, rs6983267, rs9297756, rs10094059, rs7818556, rs1992833, rs986472, rs12552397, rs4273907, rs4237185, rs753032, rs11253002, rs2386841, rs10795841, rs10508422, rs7075945, rs10508678, rs539357, rs10826398, rs3818714, rs7090755, rs10993994, rs4382847, rs1891158, rs10887926, rs10788160, rs6579002, rs10832514, rs7358335, rs1944047, rs3019779, rs10896437, rs12793759, rs7106762, rs7102758, rs2449600, rs585197, rs2509867, rs11568818, rs7125415, rs11601037, rs11222496, rs4570588, rs6489721, rs3213764, rs17395631, rs4423250, rs11168936, rs10875943, rs3759129, rs902774, rs1827611, rs4760442, rs11610799, rs6539333, rs11067228, rs7485441, rs6489794, rs4119478, rs17070292, rs2293710, rs17256058, rs1950198, rs2331780, rs7141529, rs12880777, rs17123359, rs785437, rs524908, rs12903579, rs7178085, rs7164364, rs896615, rs11634741, rs9972541, rs12594014, rs11631109, rs1558902, rs8044335, rs2738571, rs885479, rs385894, rs684232, rs4925094, rs17138478, rs11649743, rs2107131, rs7213769, rs12946864, rs306801, rs138213197, rs1863610, rs17224342, rs9911515, rs12947919, rs966304, rs17744022, rs7234917, rs1943821, rs2227270, rs1363120, rs888663, rs1227732, rs1054564, rs4806120, rs11672691, rs758643, rs3745233, rs6509345, rs2659051, rs2735839, rs1354774, rs2691274, rs6090461, rs2297434, rs6062509, rs2315654, rs2823118, rs2838053, rs398146, rs16988279, rs2269640, rs4822763, rs132774, rs747745, rs5978944, rs6530238, rs5934705, rs5935063, rs4830488, rs17318620, rs5945619, rs5945637, rs11091768, rs2473057, rs5918762, rs4844228, rs6625760 and rs17324573 (list 2); and/or rs138213197, rs7818556, rs6983267, rs10993994, rs12793759, rs16901979, rs9911515, rs1016343, rs7106762, rs6579002, rs16860513, rs5945619, rs16902094, rs10896437, rs651164, rs7679673, rs13265330, rs2047408, rs10107982, rs620861, rs9297746, rs1992833, rs7213769, rs2710647, rs888507, rs17021918, rs12500426, rs2028900, rs7102758, rs16901922, rs6062509, rs2659051, rs17832285, rs12543663, rs4699312, rs11091768, rs3120137, rs6794467, rs10086908, rs7141529, rs2315654, rs12151618, rs747745, rs1009, rs2132276, rs2735839, rs11568818, rs684232, rs9364554, rs9830294, rs2660753, rs10807843, rs1933488, rs17467139, rs12947919, rs721048, rs385894, rs2331780, rs1894292, rs2107131, rs6545962, rs11649743, rs758643, rs2297434, rs902774, rs2647262, rs17224342, rs5918762, rs11672691, rs17138478, rs3019779, rs1873555, rs9457937, rs2838053, rs12946864, rs12475433, rs3765065, rs2018334, rs3771570, rs4871779, rs10875943, rs11601037, rs6489721, rs11168936, rs9297756, rs11900952, rs6569371, rs7752029, rs5934705, rs3745233, rs1482679, rs749264, rs6625760, rs5978944, rs2366711, rs5935063, rs10199796, rs2473057, rs4925094, and rs3096702 (list 3);

and/or

rs10086908, rs1009, rs10094059, rs10107982, rs1016343, rs10178804, rs10199796, rs10807843, rs10875943, rs10896437, rs10993994, rs11091768, rs11168936, rs11568818, rs11601037, rs11649743, rs11900952, rs12151618, rs12475433, rs12490248, rs12500426, rs12543663, rs12793759, rs12946864, rs12947919, rs13265330, rs138213197, rs1482679, rs16860513, rs16901841, rs16901922, rs16901979, rs16902094, rs17021918, rs17138478, rs17224342, rs17467139, rs1873555, rs1894292, rs1933488, rs1992833, rs2025645, rs2028900, rs2047408, rs2107131, rs2132276, rs2270785, rs2297434, rs2315654, rs2331780, rs2366711, rs2465796, rs2473057, rs2659051, rs2660753, rs2710647, rs2735839, rs2823118, rs3019779, rs306801, rs3096702, rs3120137, rs3745233, rs3765065, rs4245739, rs4699312, rs4871779, rs4925094, rs5918762, rs5934705, rs5935063, rs5945619, rs5978944, rs6062509, rs6090461, rs620861, rs6489721, rs651164, rs6545962, rs6569371, rs6579002, rs6625760, rs6794467, rs684232, rs6983267, rs7102758, rs7106762, rs7213769, rs747745, rs749264, rs758643, rs7679673, rs7752029, rs7818556, rs888507, rs902774, rs9297746, rs9297756, rs9359428, rs9364554, and rs9911515 (list 4), or a subset of any one of lists 1-4, said subset comprising about 90%, 80%, 75% or 75% of the SNPs of any one of the lists 1-4;

and

wherein a defined amount of a PCa related biomarker(s) comprises one or more kallikrein-like PCa biomarker(s) and wherein at least one of the kallikrein-like PCa biomarkers is selected from the group consisting of (i) PSA, (ii) total PSA (tPSA), (iii) free PSA (fPSA), and (iv) hK2.

There is also provided a method wherein in step b), it is furthermore:

i. determined a presence or concentration of a defined amount of PCa related biomarker(s) in a biological sample obtained from the individual of said PCaGS;

ii. determined a PCa related genetic status by determining a presence or absence of a defined amount of one or more risk alleles of a SNP(s) related to PCa in said PCaGS individual;

iii. combined data from said individual regarding said presence or concentration of a defined amount of PCa related biomarker(s), and data from said individual regarding a PCa related genetic status to form a PCaGS composite value;

iv. correlated said PCaGS composite value to the presence or non-presence of PCa in said individual by comparing the composite value to a pre-determined cut-off value established with control samples of a known PCaGS and control samples of non-presence of PCa.

In step a) of a method disclosed herein an individual is determined to belong to a PCaGS if said individual is a homozygote risk allele carrier of one or more SNP(s) with an odds ratio from about 1.2 to 2 and/or a heterozygote risk allele carrier of one or more SNP(s) with an odds ratio of >2. More specifically, said one or more SNP(s) is selected from the group consisting of rs16901979, rs7818556, rs12793759 and rs138213197. Furthermore, there is provided a method wherein an individual is determined to belong to a PCaGS if said individual is a heterozygote risk allele carrier of two or more different SNP(s) each with an odds ratio from 1.2 to 2, wherein said SNPs are selected from the group consisting of rs16901979, rs7818556, rs12793759 and rs138213197.

There is also provided a method herein, wherein in step a) an individual is determined to belong to a PCaGS if said individual carries at least one risk allele of rs138213197. There is also provided a method herein, wherein in step a) an individual is determined to belong to a PCaGS if said individual has a genetic risk score exceeding a threshold value, wherein said genetic risk score is based on one or more SNP(s) selected from the group consisting of rs16901979, rs7818556, rs12793759, rs138213197, rs16860513, and rs7106762.

In another aspect, there is provided the use of an assay device for performing a method as disclosed herein, said assay device comprising a solid phase having immobilised thereon at least three different categories of ligands, wherein:

- the first category of said ligands binds specifically to a defined amount of PCa related biomarker(s), as defined herein, and includes a plurality of different ligands binding specifically to each of said PCa related biomarker(s), and
- the second category of said ligands binds specifically to a defined amount of SNP(s) related to PCa, as defined herein, or a subset thereof, and includes a plurality of different ligands binding specifically to each of said SNPs, and
- the third category of said ligands binds specifically to said PCaGS SNP(s).

There is in a further aspect of the present invention provided the use of an assay device for performing a method as described herein, wherein said assay device is present in a test kit, said test kit further comprising one or more detection molecules for specifically detecting the PCa related biomarker(s), the SNP(s) related to PCa and the PCa Genetic Subpopulation (PCaGS) SNP(s) bound to said first, second and third category of ligands, respectively.

In yet another aspect of the invention, there is provided a computer program product directly loadable into the internal memory of a digital computer, characterized in that said computer program comprises software code means for performing at least steps c) iii) and c) iv) of a method defined herein and/or steps iii) and iv) of another method as defined herein.

In yet another aspect, there is provided a computer program comprising computer-executable instructions for causing a computer, when the computer-executable instructions are executed on a processing unit comprised in the computer, to perform at least steps c) iii) and c) iv) of a method as defined herein.

There is also provided a computer program product comprising a computer-readable storage medium, the computer-readable storage medium having the computer program as mentioned herein embodied therein.

There is also provided herein a data processing apparatus or device comprising means for carrying out at least steps c) iii) and c) iv) of a method as defined herein and/or steps iii) and iv) of another method as defined herein.

There is also provided an apparatus comprising an assay device as defined herein and a computer program product.

BRIEF DESCRIPTION OF THE DRAWINGS

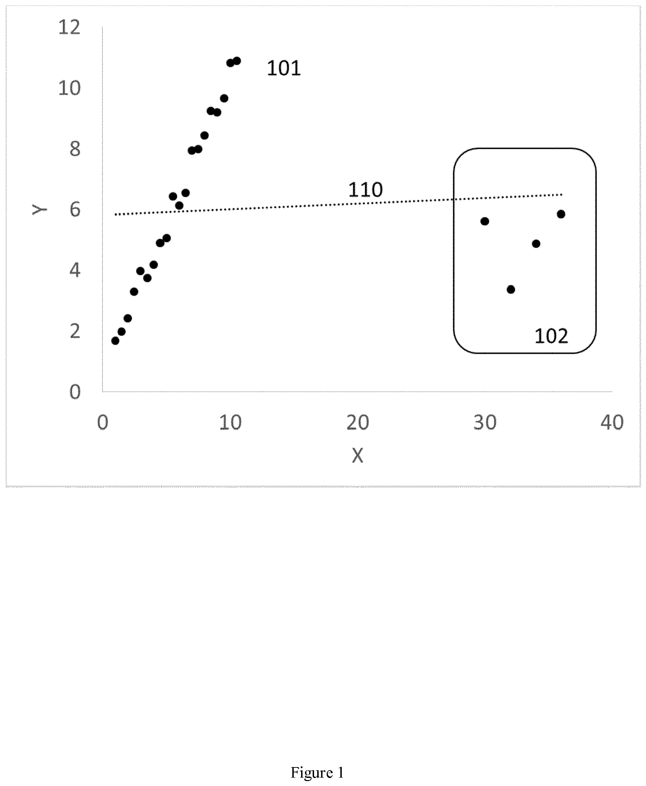

Figure 1 shows a hypothetical illustration for the rationale of special handling of a subpopulation, e.g. a PCaGS as defined herein.

DETAILED DESCRIPTION OF THE INVENTION

For the purpose of this application and for clarity, the following definitions are made:

The term "PSA" refers to serum prostate specific antigen in general. PSA exists in different forms, where the term "free PSA" refers to PSA that is unbound or not bound to another molecule, the term "bound PSA" refers to PSA that is bound to another molecule, and finally the term "total PSA" refers to the sum of free PSA and bound PSA. The term "F/T PSA" is the ratio of unbound PSA to total PSA. There are also molecular derivatives of PSA, where the term "proPSA" refers to a precursor inactive form of PSA and "intact PSA" refers to an additional form of proPSA that is found intact and inactive.

The term "diagnostic assay" refers to the detection of the presence or nature of a pathologic condition. It may be used interchangeably with "diagnostic method". Diagnostic assays differ in their sensitivity and specificity.

One measure of the usefulness of a diagnostic tool is "area under the receiver - operator characteristic curve", which is commonly known as ROC-AUC statistics. This widely accepted measure takes into account both the sensitivity and specificity of the tool. The ROC-AUC measure typically ranges from 0.5 to 1.0, where a value of 0.5 indicates the tool has no diagnostic value and a value of 1.0 indicates the tool has 100% sensitivity and 100% specificity.

The term "sensitivity" refers to the proportion of all subjects with PCa or aPCa that are correctly identified as such (which is equal to the number of true positives divided by the sum of the number of true positives and false negatives).

The term "specificity" refers to the proportion of all subjects healthy with respect to PCa (i.e. not having PCa) that are correctly identified as such (which is equal to the number of true negatives divided by the sum of the number of true negatives and false positives).

The term "biomarker" refers to a protein, a part of a protein, a peptide or a polypeptide, which may be used as a biological marker, e.g. for diagnostic purposes.

The term "kallikrein-like biomarker" refers to protein biomarkers belonging to or being related to the kallikrein family of proteins, including but not limited to Prostate-specific antigen (PSA) in either free form or complexed form, pro PSA (a collection of isoforms of PSA) and in particular the truncated form (-2) pro PSA, intact PSA, human prostatic acid phosphatase (PAP), and human kallikrein 2 (hK2).

The term "single nucleotide polymorphisms" (SNP) refer to the genetic properties of a defined locus in the genetic code of an individual. An SNP can be related to increased risk for PCA, and can hence be used for diagnostic or prognostic assessments of an individual. The Single Nucleotide Polymorphism Database (dbSNP) is an archive for genetic variation within and across different species developed and hosted by the National Center for Biotechnology Information (NCBI) in collaboration with the National Human Genome Research Institute (NHGRI), both located in the US. Although the name of the database implies a collection of one class of polymorphisms only (i.e., single nucleotide polymorphisms (SNP)), it in fact contains a range of molecular variation. Every unique submitted SNP record receives a reference SNP ID number ("rs#"; "refSNP cluster"). In this application, SNP are mainly identified using rs# numbers.

The term "aggressive prostate cancer" (aPCa) refers to a more serious condition than the average prostate cancer disease. aPCa can be defined in different ways, including but not limited to (a) prostate cancer of Gleason Score 7 or higher, (b) prostate cancer in tumor stage three or greater, (c) prostate cancer in an individual having a PSA value greater than 10 ng/mL, (d) an individual having an increasing PSA value (doubling time less than one year), and (e) computer assisted image analysis (e.g. positron emission tomography (PET) or single photon emission computerized tomography (SPECT) or computerized x-ray tomography (CT) or magnetic resonance imaging (MRI) or ultrasound imaging or any other computer assisted image analysis) indicating a tumor size in the higher quartile of the patient population.

The term "medical history" refers to information related to historic examinations, diagnoses and / or therapy for any cancer disease. One non-limiting example of medical history is if a subject has been examined for the presence of PCa previously through biopsy of the prostate.

The term "composite value" refers to the combination of data related to a parameter category into a representative value for said parameter category. A composite value can typically be described as a set of equations, wherein the different equations are applicable for cases where measurement results for different subsets of the members of the parameter category is available. One non-limiting example of a method to form a composite value for a particular parameter category is to use the average of the available results for the members of said category. Herein, the terms "General PCa population composite value" as well as "PCaGS composite value" are also used to further define the composite values that are obtained when prepared from data originating from the respective subgroup and the general population.

The term "PCaGS" used herein is an abbreviation for a Prostate Cancer Genetic Subpopulation. This term is intended herein to refer to a subpopulation of individuals defined by their genetic property/properties, wherein the genetic property is a property that is deviating from the "general" prostate cancer population. A PCaGS subgroup may be generically defined as a subgroup identified through particular genetic characteristics related to a small number, such as less than 10, such as 1, 2, 3, 4, 5, 6, 7, 8, 9, or 10, of defined genetic alterations, such as one or more defined risk allele(s) of an SNP(s), wherein each defined genetic alteration alone or multiple defined genetic alterations in combination determine if an individual is member of the PCaGS subgroup. A non-limiting example of a suitable "PCaGS" is individuals that carry at least one risk allele of rs138213197. Another non-limiting example of a suitable "PCaGS" is individuals that carry more than one, such as two SNP that in concert elevates the overall risk for getting PCa at least 20%. A "PCaGS" typically accounts for less than 20% of the complete population and more often less than 10% of the population. An example of a PCaGS are individuals having one or two genetic proper(ties) individually or together known to increase or decrease the risk for PCa with more than 20%. Hence, as mentioned above commonly, a few SNP (such as 1, 2, 3, 4, or 5 SNP) are often sufficient to accurately determining membership of a particular PCaGS. In some cases, a larger number of SNP may be envisaged such as 5-10, or 10-15, or even 15-30 SNP, depending on the complexity of the defined PCaGS. The present invention relates to a PCaGS defined by the group of SNPs consisting of rs16901979, rs7818556, rs12793759 and rs138213197.

Herein, an advantage has been proven if individuals belonging to such a subpopulation (PCaGS) are handled separately in a method for indicating the presence or non-presence of prostate cancer or aggressive prostate cancer in such an individual. This means that for such a group other criteria and/or cut-off values are preferably used for indicating a presence or non-presence of prostate cancer or aggressive prostate cancer than for individuals belonging to the "general" prostate cancer population, as further defined herein. If these differences are not taken into account, there is a risk that the method produces higher quantities of false positive and/or false negative results for either the subgroup or the general population or both.

A "PCa related parameter" is intended herein to refer broadly to any further parameter that is determined in an individual, who either belongs to a PCaGS or who does not belong to a PCaGS, and that improves a method for indicating a presence or non-presence of prostate cancer or aggressive prostate cancer as defined herein. For the individual belonging to the PCaGS, this may e.g. mean that a PSA value is measured and handled in a PCaGS population dependent manner as defined herein (i.e. using different cut-off values than for the general PCa population) and/or that data regarding a PCa related biomarker status and a PCa related genetic status is combined into a composite value and compared with specific PCaGS defined cut-off values to indicate the presence or non-presence of prostate cancer or aggressive prostate cancer.

A "genetic analysis" performed in a method herein refers to the determination of a genetic property, more particularly the determination of the presence or non-presence of one or more defined risk alleles for prostate cancer or aggressive prostate cancer in an individual. This genetic analysis may also be referred to herein as determining a "PCaGS related genetic status". The "genetic analysis" step may also include the step to determine a PCa related genetic status by determining a presence of a defined amount of SNP(s) related to PCa in said individual, as further defined herein.

The term "parameter category" refers to a group or a family of related parameters, such as related biomarkers or related SNPs, which are partly or completely redundant in terms of predictive performance. One example of a parameter category is "kallikrein-like biomarkers", a category which includes for example PSA, total PSA (tPSA), intact PSA (iPSA), free PSA (fPSA), and hk2. In the prediction models of the present invention, it may be sufficient to have measurement results (data) for a subset of the members of each category, so as to make each category represented in the prediction model, albeit using only a subset of the members of the respective categories. The term "parameter category" is sometimes referred to as only "category" in the present application.

The term "redundantly designed combination of data" refers to a combination of data obtained by a plurality of measurements, to form a composite value for one or more parameter categories or subsets thereof, wherein the combination of data is performed such that a composite value representing one parameter category can be produced based either on a subset of data for said category, e.g. where some data are missing or erroneous, or on the full set of data for said category.

When a "defined amount" is referred to herein, this generally concern an amount of PCa related biomarkers and/or SNP(s) related to PCa used in a method or a device presented herein. A defined amount can be any amount or concentration (for example expressed as ng/ml) of PCa related biomarkers in a biological (blood) sample. A defined amount can be any amount or number of alleles for a particular SNP(s) which enable estimating the PCa status of an individual. More specific examples of defined amounts of SNP(s) and PCa related biomarker(s) are also presented herein.

*Detailed description*

The present invention provides diagnostic methods to aid in estimating, detecting and/or determining the presence or non-presence of prostate cancer (PCa) or aggressive prostate cancer (aPCa) in a subject. The present invention is tailored to a defined subpopulation (PCaGS - Prostate Cancer Genetic Subpopulation) in order to increase the performance and the usefulness of the invention within said subpopulation. Even though the present invention can be applied to the general population of male individuals, it is possible to construct diagnostic methods for the detection of PCa or aPCa with enhanced performance for the defined subpopulations. Accordingly, the essence of the invention and what has surprisingly been shown herein is the fact that certain individuals, without wishing to be bound by theory, appears to have a certain biology which makes diagnostic methods and cut-off values applicable to the general population not equally suitable for an individual belonging to a subpopulation. Herein, this is defined as a genetic subpopulation (PCaGS).

The present invention utilizes the differences in this subpopulation to improve previous methods for indicating a presence or non-presence of prostate cancer or aggressive prostate cancer, particularly methods utilizing a PCa genetic and biomarker status for said indication.

Individuals having at least one single genetic property known to increase or decrease the risk for PCa with more than 20% are examples of a subpopulation PCaGS. Within the first mentioned subpopulation, individuals are at greater risk for PCa or aPCa than the general population, which has been previously shown, for example in "Germline mutations in HOXB13 and prostate-cancer risk." by Ewing CM and co-authors as published in N Engl J Med. 2012 Jan 12;366(2): 141-9. The fact that one single genetic mutation results in a large increase in risk means that the mutation has a critical negative impact on the biological machinery. In such cases, without wishing to be bound by theory, it is possible that biology as such becomes different in respect to other biomarkers, which in turn would mean that the traditional limits and ranges for biomarkers may not be valid for the subpopulation PCaGS. This means that it is beneficial to handle members of the subpopulation PCaGS in a different manner, as further described herein, than the general population when assessing if an individual is at elevated risk for having PCa or aPCa.

Accordingly, there is provided herein a method for indicating a presence or non-presence of a prostate cancer (PCa) in an individual, said method comprising the steps of:

a) performing a genetic analysis of a biological sample obtained from said individual comprising determining a presence or non-presence of risk alleles of a group of Single Nucleotide Polymorphism (SNP) related to a PCa Genetic Subpopulation (PCaGS), wherein if at least one of said risk alleles is/are present in said sample, said individual is determined to belong to said PCaGS, and if said one or more defined risk allele(s) of a SNP is not present in said sample, said individual is determined not to belong to said PCaGS, wherein said group of SNPs consists of rs16901979, rs7818556, rs12793759 and rs138213197;

b) if in step a) said individual is determined to belong to a PCaGS, then determine and characterize one or more additional PCa related parameter(s) in said individual to indicate a presence or a non-presence of PCa in said PCaGS individual, wherein said one or more additional PCa related parameter(s) in said individual comprises measuring a total PSA value in said individual;

c) if said individual in step a) is determined not to belong to a PCaGS, then

i) determine a presence or concentration of a defined amount of PCa related biomarker(s) in said individual;
ii) determine a PCa related genetic status by determining a presence or absence of a defined amount of risk allele(s) of a SNP(s) related to PCa in said individual;
iii) combine data from said individual regarding said presence or concentration of a defined amount of PCa related biomarker(s), and data from said individual regarding a PCa related genetic status to form a general PCa population composite value;

d) correlate said general PCa population composite value to the presence or non-presence of PCa in said individual by comparing the general PCa population composite value to a pre-determined cut-off value established with control samples of known general PCa population and control samples of non-presence of PCa, wherein said defined amount of one or more risk allele(s) of an SNP related to PCa comprises SNPs present in any one of the following lists:

rs138213197, rs7818556, rs6983267, rs10993994, rs12793759, rs16901979, rs9911515, rs1016343, rs7106762, rs6579002, rs16860513, rs5945619, rs16902094, rs10896437, rs651164, rs7679673, rs13265330, rs2047408, rs10107982, rs620861, rs9297746, rs1992833, rs7213769, rs2710647, rs888507, rs17021918, rs12500426, rs2028900, rs7102758, rs16901922, rs6062509, rs2659051, rs12543663, rs4699312, rs11091768, rs3120137, rs6794467, rs10086908, rs2315654, rs12151618, rs747745, rs1009, rs2132276, rs2735839, rs11568818, rs684232, rs9364554, rs2660753, rs10807843, rs1933488, rs17467139, rs12947919, rs2331780, rs1894292, rs2107131, rs6545962, rs11649743, rs758643, rs2297434, rs902774, rs17224342, rs5918762, rs17138478, rs3019779, rs1873555, rs12946864, rs12475433, rs3765065, rs4871779, rs10875943, rs1 1601037, rs6489721, rs11168936, rs9297756, rs11900952, rs6569371, rs7752029, rs5934705, rs3745233, rs1482679, rs749264, rs6625760, rs5978944, rs2366711, rs5935063, rs10199796, rs2473057, rs4925094, rs3096702, rs12490248, rs4245739, rs10094059, rs306801, rs2823118, rs2025645, rs9359428, rs10178804, rs6090461, rs2270785 , rs16901841, and rs2465796 (list 1);
and/or
rs582598, rs439378, rs2207790, rs1046011, rs10458360, rs7525167, rs10489871, rs7529518, rs4245739, rs4512641, rs10178804, rs11900952, rs1873555, rs10191478, rs6755901, rs6545962, rs721048, rs2710647, rs12612891, rs2028900, rs1009, rs12233245, rs6760417, rs10496470, rs10199796, rs12475433, rs16860513, rs12151618, rs3765065, rs13017302, rs12988652, rs871688, rs749264, rs3771570, rs4346531, rs6770955, rs12637074, rs2660753, rs13319878, rs6437715, rs2162185, rs1515542, rs2270785, rs9830294, rs1439024, rs6762443, rs888507, rs6794467, rs12490248, rs1477886, rs4833103, rs3796547, rs17779822, rs2366711, rs16849146, rs1894292, rs12640320, rs3805284, rs12500426, rs4699312, rs17021918, rs7679673, rs2047408, rs2647262, rs12506850, rs7658048, rs2078277, rs12505546, rs13113975, rs4246742, rs2736098, rs401681, rs11134144, rs10060513, rs40485, rs2087724, rs1482679, rs16901841, rs1295683, rs2070874, rs7752029, rs2018334, rs9358913, rs1140809, rs409558, rs3096702, rs9267911, rs2025645, rs9359428, rs6569371, rs2813532, rs1933488, rs712242, rs6934898, rs9456490, rs651164, rs3120137, rs9364554, rs9457937, rs10486562, rs10807843, rs7801918, rs6962297, rs2465796, rs6957416, rs7777631, rs2272316, rs6961773, rs2132276, rs13265330, rs16887736, rs2911756, rs2272668, rs2339654, rs1380862, rs9297746, rs12543663, rs10086908, rs16901922, rs1016343, rs17832285, rs16901979, rs4871779, rs10107982, rs16902094, rs620861, rs17467139, rs6983267, rs9297756, rs10094059, rs7818556, rs1992833, rs986472, rs12552397, rs4273907, rs4237185, rs753032, rs11253002, rs2386841, rs10795841, rs10508422, rs7075945, rs10508678, rs539357, rs10826398, rs3818714, rs7090755, rs10993994, rs4382847, rs1891158, rs10887926, rs10788160, rs6579002, rs10832514, rs7358335, rs1944047, rs3019779, rs10896437, rs12793759, rs7106762, rs7102758, rs2449600, rs585197, rs2509867, rs11568818, rs7125415, rs11601037, rs11222496, rs4570588, rs6489721, rs3213764, rs17395631, rs4423250, rs11168936, rs10875943, rs3759129, rs902774, rs1827611, rs4760442, rs11610799, rs6539333, rs11067228, rs7485441, rs6489794, rs4119478, rs17070292, rs2293710, rs17256058, rs1950198, rs2331780, rs7141529, rs12880777, rs17123359, rs785437, rs524908, rs12903579, rs7178085, rs7164364, rs896615, rs11634741, rs9972541, rs12594014, rs11631109, rs1558902, rs8044335, rs2738571, rs885479, rs385894, rs684232, rs4925094, rs17138478, rs11649743, rs2107131, rs7213769, rs12946864, rs306801, rs138213197, rs1863610, rs17224342, rs9911515, rs12947919, rs966304, rs17744022, rs7234917, rs1943821, rs2227270, rs1363120, rs888663, rs1227732, rs1054564, rs4806120, rs11672691, rs758643, rs3745233, rs6509345, rs2659051, rs2735839, rs1354774, rs2691274, rs6090461, rs2297434, rs6062509, rs2315654, rs2823118, rs2838053, rs398146, rs16988279, rs2269640, rs4822763, rs132774, rs747745, rs5978944, rs6530238, rs5934705, rs5935063, rs4830488, rs17318620, rs5945619, rs5945637, rs11091768, rs2473057, rs5918762, rs4844228, rs6625760 and rs17324573 (list 2); and/or
rs138213197, rs7818556, rs6983267, rs10993994, rs12793759, rs16901979, rs9911515, rs1016343, rs7106762, rs6579002, rs16860513, rs5945619, rs16902094, rs10896437, rs651164, rs7679673, rs13265330, rs2047408, rs10107982, rs620861, rs9297746, rs1992833, rs7213769, rs2710647, rs888507, rs17021918, rs12500426, rs2028900, rs7102758, rs16901922, rs6062509, rs2659051, rs17832285, rs12543663, rs4699312, rs11091768, rs3120137, rs6794467, rs10086908, rs7141529, rs2315654, rs12151618, rs747745, rs1009, rs2132276, rs2735839, rs11568818, rs684232, rs9364554, rs9830294, rs2660753, rs10807843, rs1933488, rs17467139, rs12947919, rs721048, rs385894, rs2331780, rs1894292, rs2107131, rs6545962, rs11649743, rs758643, rs2297434, rs902774, rs2647262, rs17224342, rs5918762, rs11672691, rs17138478, rs3019779, rs1873555, rs9457937, rs2838053, rs12946864, rs12475433, rs3765065, rs2018334, rs3771570, rs4871779, rs10875943, rs11601037, rs6489721, rs11168936, rs9297756, rs11900952, rs6569371, rs7752029, rs5934705, rs3745233, rs1482679, rs749264, rs6625760, rs5978944, rs2366711, rs5935063, rs10199796, rs2473057, rs4925094, and rs3096702 (list 3);
and/or
rs10086908, rs1009, rs10094059, rs10107982, rs1016343, rs10178804, rs10199796, rs10807843, rs10875943, rs10896437, rs10993994, rs11091768, rs11168936, rs11568818, rs11601037, rs11649743, rs11900952, rs12151618, rs12475433, rs12490248, rs12500426, rs12543663, rs12793759, rs12946864, rs12947919, rs13265330, rs138213197, rs1482679, rs16860513, rs16901841, rs16901922, rs16901979, rs16902094, rs17021918, rs17138478, rs17224342, rs17467139, rs1873555, rs1894292, rs1933488, rs1992833, rs2025645, rs2028900, rs2047408, rs2107131, rs2132276, rs2270785, rs2297434, rs2315654, rs2331780, rs2366711, rs2465796, rs2473057, rs2659051, rs2660753, rs2710647, rs2735839, rs2823118, rs3019779, rs306801, rs3096702, rs3120137, rs3745233, rs3765065, rs4245739, rs4699312, rs4871779, rs4925094, rs5918762, rs5934705, rs5935063, rs5945619, rs5978944, rs6062509, rs6090461, rs620861, rs6489721, rs651164, rs6545962, rs6569371, rs6579002, rs6625760, rs6794467, rs684232, rs6983267, rs7102758, rs7106762, rs7213769, rs747745, rs749264, rs758643, rs7679673, rs7752029, rs7818556, rs888507, rs902774, rs9297746, rs9297756, rs9359428, rs9364554, and rs9911515 (list 4), or a subset of any one of lists 1-4, said subset comprising about 90%, 80%, 75% or 70% of the SNPs of any one of the lists 1-4; and

wherein a defined amount of a PCa related biomarker(s) comprises one or more kallikrein-like PCa biomarker(s) and wherein at least one of the kallikrein-like PCa biomarkers is selected from the group consisting of (i) PSA, (ii) total PSA (tPSA), (iii) free PSA (fPSA), and (iv) hK2.

There is furthermore provided a method herein, wherein in step b), it is furthermore:

i. determined a presence or concentration of a defined amount of PCa related biomarker(s) in a biological sample obtained from the individual of said PCaGS;
ii. determined a PCa related genetic status by determining a presence or absence of a defined amount of one or more risk allele(s) of a SNP(s) related to PCa in said PCaGS individual;
iii. combined data from said individual regarding said presence or concentration of a defined amount of PCa related biomarker(s), and data from said individual regarding a PCa related genetic status to form a PCaGS composite value;
iv. correlate said PCaGS composite value to the presence or non-presence of PCa in said individual by comparing the composite value to a pre-determined cut-off value established with control samples of a known PCaGS and control samples of non-presence of PCa.

The methods provided herein may be used for indicating the presence or non-presence of prostate cancer or aggressive prostate cancer. When the purpose of the method is to indicate the presence or non-presence of prostate cancer, a cut-off value may be established with samples obtained from a known general PCa population, with control samples of a known PCaGS and control samples of non-presence of PCa. When the purpose of the method is to indicate the presence or non-presence of aggressive prostate cancer, a cut-off value may be established with samples of known general aggressive PCa population, with control samples of a known aggressive PCaGS and control samples of non-presence of PCa.

In step a) of said method, an individual is determined to belong to a PCaGS if said individual is a homozygote risk allele carrier of one or more SNP(s) with an odds ratio from 1.2 to 2 and/or a heterozygote risk allele carrier of one or more SNP(s) with an odds ratio of >2. More specifically, said one or more SNP(s) qualifying for such a definition are selected from the group consisting of rs16901979, rs7818556, rs12793759 and rs138213197.

In addition, in step a) an individual may be determined to belong to a PCaGS if said individual is a heterozygote risk allele carrier of two or more different SNP(s), each SNP with an odds ratio from 1.2 to 2, wherein said SNPs are selected from the group consisting of rs16901979, rs7818556, rs12793759 and rs138213197.

Additional SNP(s) are presented in Table 1 in the below. SNP(s) listed in Table 1 have been assigned an Odds Ratio greater than 1.2 in at least one cohort, but may have been assigned an Odds Ratio less than 1.2 in other cohorts. Hence, all SNP(s) in Table 1 are suitable candidates to define a PCaGS. However, herein, a PCaGS according to the present invention is defined by rs16901979, rs7818556, rs12793759 and rs138213197.

Table 1: SNPs odds ratio.

| SNP | $Or_{max}$ | SNP | $Or_{max}$ |
|---|---|---|---|
| rs12947919 | 1.201 | rs7125415 | 1.25 |
| rs721048 | 1.205 | rs1016343 | 1.266 |
| rs2911756 | 1.21 | rs6579002 | 1.27 |
| rs17467139 | 1.214 | rs2162185 | 1.275 |
| rs7164364 | 1.214 | rs885479 | 1.29 |
| rs132774 | 1.218 | rs2660753 | 1.316 |
| rs12637074 | 1.22 | rs16887736 | 1.317 |
| rs539357 | 1.22 | rs12793759 | 1.339 |
| rs6962297 | 1.227 | rs7818556 | 1.43 |
| rs 16902094 | 1.228 | rs2659051 | 1.463 |

(continued)

| SNP | Or$_{max}$ | SNP | Or$_{max}$ |
|---|---|---|---|
| rsl0489871 | 1.235 | rs2735839 | 1.627 |
| rsl1091768 | 1.24 | rs16901979 | 1.736 |
| rs12490248 | 1.244 | rs138213197 | 3.5 |
| rsl0993994 | 1.247 | | |

Furthermore, an individual may be determined to belong to a PCaGS if said individual carries at least one single genetic property known to increase risk for PCa of more than 20%, or if said individual carries two genetic properties that together increase the risk for prostate cancer with more than 20%. Herein, such SNPs are rs16901979, rs7818556, rs12793759 and rs138213197.

Examples of genetic alterations that are associated with approximately 20% increase of risk (or more) for PCa or aPCa include, but are not limited to rs16901979, rs7818556, rs12793759 and rs138213197. There are also examples of genetic alterations which results in a significant reduction of risk, such as rs16860513 and rs7106762 to mention two non-limiting examples. Further examples are mentioned in Table 2 below. SNP(s) listed in Table 2 have been assigned an Odds Ratio less than 0.8 in at least one cohort, but may have been assigned an Odds Ratio greater than 0.8 in other cohorts. Hence, all SNP(s) in Table 2 are suitable candidates to define a PCaGS. Such SNPs do not define the PCaGS according to the present invention.

Table 2: SNPs odds ratios.

| SNP | Or$_{min}$ | SNP | Or$_{min}$ |
|---|---|---|---|
| rsl6860513 | 0.5892 | rs3765065 | 0.7777 |
| rs5945637 | 0.64 | rs6983267 | 0.7833 |
| rs7090755 | 0.7604 | rs17832285 | 0.7941 |
| rs11568818 | 0.7619 | rs12151618 | 0.7981 |
| rs888507 | 0.7676 | rs7752029 | 0.7996 |

There is also provided a method, wherein in step a) an individual is determined to belong to a PCaGS if said individual carries at least one risk allele of rs138213197. This one risk allele is associated with an increased risk for PCa of more than 20%. There is also provided a method wherein in step a) an individual is determined to belong to a PCaGS if said individual has a (PCaGS) genetic risk score exceeding a threshold value, wherein said genetic risk score is based on one or more SNP(s) selected from the group consisting of rs16901979, rs7818556, rs12793759, rs138213197, rs16860513, and rs7106762.

In further aspects, there is provided a method, wherein a PSA value is measured in said biological sample obtained from said individual in step b) ii) and wherein the PSA cut-off value for indicating a presence of prostate cancer in a PCaGS individual is significantly lower than a standard general population PSA cut-off value for indicating a presence of prostate cancer.

"Significantly lower" in this regard may e.g. be at least about 10% lower than a standard cut-off PSA value, such as at least about 10%, 15%, 20%, 30%, 40% or even 50% lower than a standard cut-off value, such as at least about 10%, 15%, 20%, 30%, 40% or even 50% lower than e.g. about 4.0 ng/ml or 3.0 ng/ml depending on region.

There is also a possibility that a "significantly higher" cut-off value should be used as compared to a standard PSA cut-off value. In this regard, a significantly higher value may be about 10% higher than a standard PSA value, such as about 10%, 15%, 20%, 30%, 40% or even 50% higher than a standard cut-off value, such as 10%, 15%, 20%, 30%, 40% or even 50% higher than e.g. about 4.0 ng/ml or about 3.0 ng/ml depending on region.

As an example, under the assumption that PSA $\geq$ 4.0 ng/ml is used as cut-off for the general population, the PSA cut-off value for said PCaGS individual, when defined as a member of the PCaGS defined by being a homozygote risk allele carrier of one or more SNP(s) with an odds ratio from 1.2 to 2 and/or a heterozygote risk allele carrier of one or more SNP(s) with an odds ratio of >2, e.g. carrying rs16901979, rs7818556, rs12793759, and/or rs138213197, may be $\geq$ about 3.6 ng/ml, and wherein a PSA value $\geq$ about 3.6 ng/ml indicates an increased risk for presence of prostate cancer or aggressive prostate cancer in said PCaGS individual from which said biological sample originates.

As previously mentioned, there is provided a method wherein in step a) an individual is determined to belong to a PCaGS if said individual carries at least one risk allele of rs138213197. There is also provided a method, wherein a PSA value is measured in said biological sample obtained from said PCaGS individual carrying at least one risk allele of rs138213197 in step b), and wherein the PSA cut-off value for indicating a presence of prostate cancer in said PCaGS individual is significantly lower, as mentioned previously herein, than a standard general population PSA cut-off value for indicating a presence of prostate cancer.

Tables 5, 6 and 7 of example 6 shows PCaGS specific PSA cut-off values to match performance of PSA = 3 ng/ml as used for a general population for detecting aggressive prostate cancer, PSA = 3 ng/mL as used for a general population for detecting prostate cancer and PSA = 4 ng/mL as used for detecting prostate cancer, respectively. Accordingly, these values can be set as alternative PSA cut-off values for detecting PCa in these particular PCaGS.

The PCaGS of example 6 comprise:

- Individuals who are HOXB13 positive, i.e. positive for rs138213197 (PCaGS_ex2)
- Individuals for which a PCaGS defining risk score containing (rs16901979, rs7818556, rs12793759, rs138213197) has a value greater than 0.7 (PCaGS_61)
- Individuals for which a subgroup defining risk score containing (rs16901979, rs7818556, rs12793759, rs138213197, rs16860513, rs7106762) has a value greater than 0.7 (PCaGS_62)
- Individuals for which a PCaGS defining risk score containing SNPs listed in Table 1 and Table 2 has a value greater than 0.45 (PCaGS_63).

As shown in example 6, to match a sensitivity or specificity of the commonly applied PSA=3ng/mL as used for the general population for detecting aggressive PCa in a PCaGS_ex2 individual, a PSA cutoff of about 2.5 to about 2.7 ng/mL and about 1.3 to about 1.5 ng/mL, respectively, would be appropriate for indicating a presence of aggressive PCa in such a PCaGS individual (Table 5). As seen in Table 6, a PSA cut-off value for said PCaGS individual (PCaGS_ex2) may be ≥ about 1.6 ng/ml, wherein a PSA value of ≥ about 1.6 ng/ml may indicate an increased risk for presence of prostate cancer or aggressive prostate cancer in said PCaGS individual from which said biological sample originates, depending on the chosen sensitivity or specificity and when matching performance of PSA = 3 ng/mL as used for general population when detecting prostate cancer. Table 7 of example 6 illustrates how the same approach may be applied to the PCa subgroups to match the commonly applied PSA value of 4 ng/mL for detecting prostate cancer in the general population. In the same manner, PSA cut-off values for the other mentioned PCaGS can be extrapolated from Tables 5 to 7 of example 9.

Accordingly, there is furthermore provided herein a method wherein the PSA cut-off value for indicating a presence of prostate cancer in a PCaGS individual is about 1.8 to about 2.0 ng/ml or about 1.3 to about 1.5 ng/mL, to match a performance of PSA of about 3 ng/mL, with regards to sensitivity and specificity, respectively, used for a general population for detecting prostate cancer. This may be applied e.g. to PCaGS identified in example 6 as PCaGS_62.

There is also provided a method, wherein the PSA cut-off value for indicating a presence of aggressive prostate cancer in said PCaGS individual is about 2.7 to about 2.9 ng/ml or about 1.4 to about 1.5 ng/mL, to match a performance of PSA of about 3 ng/mL, with regards to sensitivity and specificity, respectively, used for a general population for detecting aggressive prostate cancer. This may be applied e.g. to PCaGS identified in example 6 as PCaGS_62.

There is also provided a method, wherein the PSA cut-off value for indicating a presence of prostate cancer in said PCaGS individual is about 1.5 to about 1.7 ng/mL or about 1.1 to about 1.3 ng/mL to match a performance of PSA of about 3 ng/mL, with regards to sensitivity and specificity, respectively, used for a general population for detecting prostate cancer. This may be applied e.g. to PCaGS identified in example 6 as PCaGS_ex2.

Referring to tables 5 to 7 of example 6, equal aspects are encompassed by the present disclosure relating to the other PCaGS mentioned in said tables.

Although the invention has mainly been described in terms of genetic status related to elevated risk, for example a point mutation of DNA that can be related to increased risk for having PCa or aPCa, the invention is also applicable for cases where the genetic status is associated to a reduced risk for having aPCa or PCa. The two SNP rs16860513 and rs7106762 represents an example of two SNP that are related to significantly reduced risk for PCa or aPCa. In such an aspect, an individual is determined to belong to a PCaGS if said individual carries at least one risk allele ofrs16860513 or rs7106762. (this embodiment is not part of the invention).

In addition, although the invention has mainly been described herein in terms of genetic status related to elevated risk for PCa or aPCa due to one or more point mutations of DNA, the invention is also applicable for cases where the genetic status is determined as deletions of multiple nucleotides in sequence, alteration of telomere length, presence or absence of one or more chromosomes, and similar larger scale genetic alterations. In such an aspect, an individual is determined to belong to a PCaGS if said individual carries deletions of multiple nucleotides in sequence, alteration of telomere length, presence or absence of one or more chromosomes, and similar larger scale genetic alterations. (this embodiment is not part of the invention)

There is also provided a method herein, wherein due to that in the first step, the PCaGS is "sorted out" from the general population, indicating the presence or non-presence of PCa or aPCa also in the general population is improved.

A basic principle for a method provided herein is the use of combinations of biomarkers and genetic information in such a manner that the combinatorial use of the assessed information about the individual improves the quality of the diagnosis.

Categories/actions/parameters that are useful in the context of the present invention are mentioned in the below. It should be noted that only some of them may also be used in a method as further defined herein, in different combinations.

*Parameters/Categories/Actions, also defined herein as "PCa related parameters"*

- Collecting the family history regarding PCa from said patient (Category HIST).
- Collecting patient physical data, such as weight, BMI, age and similar (Category PPD)
- Obtaining a number of biological samples from said patient.
- In said biological samples, quantifying the presence or concentration of a plurality (defined amount) of biomarkers (Category Biomarker).
- In said biological samples, quantifying the genetic status of said patients with respect to a plurality (defined amount) of SNPs related to PCa (Category SNPpc).
- Determining if said patient belongs to the subpopulation PCaGS.
- Combining data, in a PCaGS dependent manner, from at least two of the categories defined above to form a PCaGS composite value for the use in the detection of early prostate cancer.
- Determining by using said PCaGS dependent composite value, alone or in combination with further data, if the patient is likely to suffer from PCa or aPCa.

Accordingly, the method further may comprise collecting the family history regarding PCa or aPCa, treatment history, and physical data from said individual; wherein said family history, treatment history and/or physical data are included in the combined data forming said composite value. Physical information regarding the patient is typically obtained through a regular physical examination wherein age, weight, height, BMI and similar physical data are collected.

In more detail, the step comprising the collection of family history includes, but is not limited to, the identification of if any closely related male family member (such as the father, brother or son of the patient) suffers or have suffered from PCa or aPCa.

Collecting biological samples from a patient includes, but is not limited to plasma, serum, DNA from peripheral white blood cells and urine. Hence herein, the biological sample may be a blood sample.

A method provided herein may comprise additional steps in case the composite value obtained in the method is greater than the cut-off value and/or to further improve the outcome of the method. The cut-off value may be any of the cut-off values as defined herein.

Accordingly, there is provided a method herein, further comprising recommending the individual for biopsy if the composite value is greater than the cut-off value. A method may also comprise recommending the individual to change dietary habits, to lose weight, to reach a BMI value below 30, to exercise regularly, and/or to stop smoking, if the composite value is greater than the cut-off value. In addition, a method may comprise collecting the family history regarding PCa, treatment history, and physical data from said individual; and wherein said family history, treatment history and/or physical data are included in the combined data forming said composite value.

The quantification of presence or concentration of biomarkers in a biological sample can be made in many different ways. One common method is the use of enzyme linked immunosorbent assays (ELISA) which uses antibodies and a calibration curve to assess the presence and (where possible) the concentration of a selected biomarker. ELISA assays are common and known in the art, as evident from the publication "Association between saliva PSA and serum PSA in conditions with prostate adenocarcinoma." by Shiiki N and co-authors, published in Biomarkers. 2011 Sep;16(6):498-503. Another common method is the use of a microarray assay for the quantification of presence or concentration of biomarkers in a biological sample. A typical microarray assay comprises a flat glass slide onto which a plurality of different capture reagents (typically an antibody) each selected to specifically capture one type of biomarker is attached in nonoverlapping areas on one side of the slide. The biological sample is allowed to contact, for a defined period of time, the area where said capture reagents are located, followed by washing the area of capture reagents. At this point, in case the sought-after biomarker was present in the biological sample, the corresponding capture reagent will have captured a fraction of the sought-after biomarker and keep it attached to the glass slide also after the wash. Next, a set of detection reagents are added to the area of capture reagents (which now potentially holds biomarkers bound), said detection reagents being capable of (i) binding to the biomarker as presented on the glass slide and (ii) producing a detectable signal (normally through conjugation to a fluorescent dye). It is typically required that one detection reagent per biomarker is added to the glass slide. There are many other methods capable of quantifying the presence or concentration of a biomarker, including, but not limited to, immunoprecipitation assays, immunofluorescense assays, radio-immuno-assays, and mass spectrometry using matrix-assisted laser desorption/ionization (MALDI), to mention a few examples.

Accordingly, the measurement of the presence or concentration of PCa biomarker(s) herein may be conducted by use of microarray technology.

The quantification of genetic status through the analysis of a biological sample typically involves MALDI mass spectrometry analysis based on allele-specific primer extensions, even though other methods are equally applicable. This applies to any type of genetic status, i.e. both SNPs related to PCa and SNPs related to biomarker expression.

Accordingly, the measurement of the presence or absence of a defined amount of SNP(s) may be conducted by use of MALDI mass spectrometry.

The determination of if an individual is belonging to the PCaGS can be conducted as part of determining PCa related genetic status. This means that no additional measurement or data input are required in order to determine if an individual belongs to the PCaGS subpopulation. This means that the step comprising performing a genetic analysis of a biological sample obtained from said individual comprising determining a presence or non-presence of one or more defined risk allele(s) of a Single Nucleotide Polymorphism (SNP) related to a PCa Genetic Subpopulation (PCaGS) to determine if said individual belongs to a PCa Genetic Subpopulation (PCaGS), also may include determining: i. a presence or concentration of a defined amount of PCa related biomarker(s) in a biological sample obtained from the individual of said PCaGS; ii. a PCa related genetic status by determining a presence of a defined amount of one or more risk allele(s) of a SNP(s) related to PCa in said PCaGS individual; iii. combining data from said individual regarding said presence or concentration of a defined amount of PCa related biomarker(s), and data from said individual regarding a PCa related genetic status to form a PCaGS composite value; iv. correlating said general population composite value to the presence or non-presence of PCa in said individual by comparing the composite value to a pre-determined cut-off value established with control samples of a known PCaGS and control samples of non-presence of PCa.

Suitable biomarkers for diagnosing PCa or aPCa include, but are not limited to, Prostate-specific antigen (PSA) in either free form or complexed form, pro PSA (a collection of isoforms of PSA) and in particular the truncated form (-2) pro PSA, intact PSA, human prostatic acid phosphatase (PAP), human kallikrein 2 (hK2), early prostate cancer antigen (EPCA), Prostate Secretory Protein (PSP94; also known as beta-microseminoprotein and MSMB), glutathione S-transferase π (GSTP1), and α-methylacyl coenzyme A racemase (AMACR). Related biomarkers, which may be useful for improving the diagnostic accuracy of the method includes Macrophage Inhibitory Cytokine 1 (MIC-1; also known as GDF-15).

Accordingly, a PCa related biomarker(s) herein, may comprises one or more kallikrein-like PCa biomarker(s) such as at least one, such as two, of the kallikrein-like PCa biomarkers selected from the group consisting of (i) PSA, (ii) total PSA (tPSA), (iii) free PSA (fPSA), and (iv) hK2.

A PCa related biomarker(s) herein, may also comprises one or more kallikrein-like PCa biomarker(s), such as at least one, such as two, of the kallikrein-like PCa biomarkers selected from the group consisting of (i) PSA, (ii) total PSA (tPSA), (iii) intact PSA (iPSA), (iv) free PSA (fPSA), and (v) hK2.

A PCa related biomarker(s) may also comprise MIC-1 and optionally other MIC-1 related biomarkers, or the biomarker MSMB and optionally other MSMB related biomarkers.

There is also provided a method as previously disclosed herein, wherein data from at least three, such as three, four or five PCa related biomarker(s), such as kallikrein-like biomarkers, are used for forming said composite value. In such a method, the method allows disregarding a subset of data of at least one of said PCa related biomarkers when forming said composite value, such as a subset of data of one, two, three, or four of said PCa biomarker(s), but wherein the data maintained from said PCa related biomarkers and used in said method is sufficient to generate a composite value. The amount of PCa related biomarkers, such as kallikrein-like biomarker(s), used in said method may be at least three, or in some cases two biomarkers.

Herein, the concentration of at least one of the biomarkers PSA, iPSA, tPSA, fPSA, MIC-1, MSMB and hK2 may be determined. The composite value may thereafter be calculated using a method in which the non-additive effect of a SNP related to a PCa biomarker concentration and the corresponding biomarker concentration is utilized.

The determination of a presence or concentration of a PCa biomarker may be conducted by the use of microarray technology, as previously mentioned herein.

*Suitable SNPs in the context of a method according to the invention*

Suitable SNPs related to PCa or aPCa which may be used in the context of the present invention include, but are not limited to:

List 1:
rs138213197, rs7818556, rs6983267, rs10993994, rs12793759, rs16901979, rs9911515, rs1016343, rs7106762, rs6579002, rs16860513, rs5945619, rs16902094, rs10896437, rs651164, rs7679673, rs13265330, rs2047408, rs10107982, rs620861, rs9297746, rs1992833, rs7213769, rs2710647, rs888507, rs17021918, rs12500426, rs2028900, rs7102758, rs16901922, rs6062509, rs2659051, rs12543663, rs4699312, rs11091768, rs3120137, rs6794467, rs10086908, rs2315654, rs12151618, rs747745, rs1009, rs2132276, rs2735839, rs11568818, rs684232, rs9364554, rs2660753, rs10807843, rs1933488, rs17467139, rs12947919, rs2331780, rs1894292, rs2107131, rs6545962, rs11649743, rs758643, rs2297434, rs902774, rs17224342, rs5918762, rs17138478, rs3019779, rs1873555, rs12946864, rs12475433, rs3765065, rs4871779, rs10875943, rs11601037, rs6489721, rs11168936, rs9297756, rs11900952, rs6569371, rs7752029, rs5934705, rs3745233, rs1482679, rs749264, rs6625760, rs5978944, rs2366711, rs5935063, rs10199796, rs2473057, rs4925094, rs3096702, rs12490248, rs4245739, rs10094059, rs306801, rs2823118, rs2025645, rs9359428, rs10178804, rs6090461, rs2270785 , rs16901841, and rs2465796;
and/or

List 2
rs582598, rs439378, rs2207790, rs1046011, rs10458360, rs7525167, rs10489871, rs7529518, rs4245739, rs4512641, rs10178804, rs11900952, rs1873555, rs10191478, rs6755901, rs6545962, rs721048, rs2710647, rs12612891, rs2028900, rs1009, rs12233245, rs6760417, rs10496470, rs10199796, rs12475433, rs16860513, rs12151618, rs3765065, rs13017302, rs12988652, rs871688, rs749264, rs3771570, rs4346531, rs6770955, rs12637074, rs2660753, rs13319878, rs6437715, rs2162185, rs1515542, rs2270785, rs9830294, rs1439024, rs6762443, rs888507, rs6794467, rs12490248, rs1477886, rs4833103, rs3796547, rs17779822, rs2366711, rs16849146, rs1894292, rs12640320, rs3805284, rs12500426, rs4699312, rs17021918, rs7679673, rs2047408, rs2647262, rs12506850, rs7658048, rs2078277, rs12505546, rs13113975, rs4246742, rs2736098, rs401681, rs11134144, rs10060513, rs40485, rs2087724, rs1482679, rs16901841, rs1295683, rs2070874, rs7752029, rs2018334, rs9358913, rs1140809, rs409558, rs3096702, rs9267911, rs2025645, rs9359428, rs6569371, rs2813532, rs1933488, rs712242, rs6934898, rs9456490, rs651164, rs3120137, rs9364554, rs9457937, rs10486562, rs10807843, rs7801918, rs6962297, rs2465796, rs6957416, rs7777631, rs2272316, rs6961773, rs2132276, rs13265330, rs16887736, rs2911756, rs2272668, rs2339654, rs1380862, rs9297746, rs12543663, rs10086908, rs16901922, rs1016343, rs17832285, rs16901979, rs4871779, rs10107982, rs16902094, rs620861, rs17467139, rs6983267, rs9297756, rs10094059, rs7818556, rs1992833, rs986472, rs12552397, rs4273907, rs4237185, rs753032, rs11253002, rs2386841, rs10795841, rs10508422, rs7075945, rs10508678, rs539357, rs10826398, rs3818714, rs7090755, rs10993994, rs4382847, rs1891158, rs10887926, rs10788160, rs6579002, rs10832514, rs7358335, rs1944047, rs3019779, rs10896437, rs12793759, rs7106762, rs7102758, rs2449600, rs585197, rs2509867, rs11568818, rs7125415, rs11601037, rs11222496, rs4570588, rs6489721, rs3213764, rs17395631, rs4423250, rs11168936, rs10875943, rs3759129, rs902774, rs1827611, rs4760442, rs11610799, rs6539333, rs11067228, rs7485441, rs6489794, rs4119478, rs17070292, rs2293710, rs17256058, rs1950198, rs2331780, rs7141529, rs12880777, rs17123359, rs785437, rs524908, rs12903579, rs7178085, rs7164364, rs896615, rs11634741, rs9972541, rs12594014, rs11631109, rs1558902, rs8044335, rs2738571, rs885479, rs385894, rs684232, rs4925094, rs17138478, rs11649743, rs2107131, rs7213769, rs12946864, rs306801, rs138213197, rs1863610, rs17224342, rs9911515, rs12947919, rs966304, rs17744022, rs7234917, rs1943821, rs2227270, rs1363120, rs888663, rs1227732, rs1054564, rs4806120, rs11672691, rs758643, rs3745233, rs6509345, rs2659051, rs2735839, rs1354774, rs2691274, rs6090461, rs2297434, rs6062509, rs2315654, rs2823118, rs2838053, rs398146, rs16988279, rs2269640, rs4822763, rs132774, rs747745, rs5978944, rs6530238, rs5934705, rs5935063, rs4830488, rs17318620, rs5945619, rs5945637, rs11091768, rs2473057, rs5918762, rs4844228, rs6625760 and rs17324573;
and/or

List 3
rs138213197, rs7818556, rs6983267, rs10993994, rs12793759, rs16901979, rs9911515, rs1016343, rs7106762, rs6579002, rs16860513, rs5945619, rs16902094, rs10896437, rs651164, rs7679673, rs13265330, rs2047408, rs10107982, rs620861, rs9297746, rs1992833, rs7213769, rs2710647, rs888507, rs17021918, rs12500426, rs2028900, rs7102758, rs16901922, rs6062509, rs2659051, rs17832285, rs12543663, rs4699312, rs11091768, rs3120137, rs6794467, rs10086908, rs7141529, rs2315654, rs12151618, rs747745, rs1009, rs2132276, rs2735839, rs11568818, rs684232, rs9364554, rs9830294, rs2660753, rs10807843, rs1933488, rs17467139, rs12947919, rs721048, rs385894, rs2331780, rs1894292, rs2107131, rs6545962, rs11649743, rs758643, rs2297434, rs902774, rs2647262, rs17224342, rs5918762, rs11672691, rs17138478, rs3019779, rs1873555, rs9457937, rs2838053, rs12946864, rs12475433, rs3765065, rs2018334, rs3771570, rs4871779, rs10875943, rs11601037, rs6489721, rs11168936, rs9297756, rs11900952, rs6569371, rs7752029, rs5934705, rs3745233, rs1482679, rs749264, rs6625760, rs5978944, rs2366711, rs5935063, rs10199796, rs2473057, rs4925094, and rs3096702;
and/or

List 4:
rs10086908, rs1009, rs10094059, rs10107982, rs1016343, rs10178804, rs10199796, rs10807843, rs10875943, rs10896437, rs10993994, rs11091768, rs11168936, rs11568818, rs11601037, rs11649743, rs11900952, rs12151618, rs12475433, rs12490248, rs12500426, rs12543663, rs12793759, rs12946864, rs12947919, rs13265330, rs138213197, rs1482679, rs16860513, rs16901841, rs16901922, rs16901979, rs16902094, rs17021918, rs17138478, rs17224342, rs17467139, rs1873555, rs1894292, rs1933488, rs1992833, rs2025645, rs2028900, rs2047408, rs2107131, rs2132276, rs2270785, rs2297434, rs2315654, rs2331780, rs2366711, rs2465796, rs2473057, rs2659051, rs2660753, rs2710647, rs2735839, rs2823118, rs3019779, rs306801, rs3096702, rs3120137, rs3745233, rs3765065, rs4245739, rs4699312, rs4871779, rs4925094, rs5918762, rs5934705, rs5935063, rs5945619, rs5978944, rs6062509, rs6090461, rs620861, rs6489721, rs651164, rs6545962, rs6569371, rs6579002, rs6625760, rs6794467, rs684232, rs6983267, rs7102758, rs7106762, rs7213769, rs747745, rs749264, rs758643, rs7679673, rs7752029, rs7818556, rs888507, rs902774, rs9297746, rs9297756, rs9359428, rs9364554, rs9911515.

A subset of the SNPs in any of the above lists of SNPs may also be used, such as a subset comprising about 90% of the SNP of any of the lists of SNPs mentioned herein, or a subset comprising about 80%, such as 75%, 70%, 65% or 60% of the SNPs in any of the lists presented herein. These may be placed on the same solid support, for example the same glass slide, for simultaneous detection in a suitable analytical instrument. The list may also contain other additional SNPs. The SNP(s) present on the respective lists may also be combined.

Suitable SNPs related to other processes than PCa include, but are not limited to rs3213764, rs1354774, rs2736098, rs401681, rs10788160, rs11067228, all being related to the expression level of PSA. It is possible to also define a parameter category as "SNP related to concentration of PSA" or "SNP related to expression level of PSA", which includes SNP related to the concentration or expression level of PSA. A subset of the members of this category would be sufficient to represent the category as such in a predictive model. The SNPs rs3213764 and rs1354774 relate particularly to the expression level of free PSA.

Suitable SNPs related to other processes than PCa further include, but are not limited to rs1363120, rs888663, rs1227732, rs1054564, all being related to the expression level of the inflammation cytokine biomarker MIC1. It is possible to define a parameter category as "SNP related to concentration of MIC1" or "SNP related to expression level of MIC1" which includes SNP related to the concentration or expression level of MIC1. A subset of the members of this category would be sufficient to represent the category as such in a predictive model.

It is possible to define a parameter category as "SNP related to PCa biomarker concentration" or "SNP related to PCa biomarker expression level" which includes SNP related to the concentration or expression level of relevant biomarkers such as Prostate-specific antigen (PSA) in either free form or complexed form, pro PSA (a collection of isoforms of PSA) and in particular the truncated form (-2) pro PSA, intact PSA, human prostatic acid phosphatase (PAP), human kallikrein 2 (hK2), early prostate cancer antigen (EPCA), Prostate Secretory Protein (PSP94; also known as beta-microseminoprotein and MSMB), glutathione S-transferase $\pi$ (GSTP1), $\alpha$-methylacyl coenzyme A racemase (AMACR), and Macrophage Inhibitory Cytokine 1 (MIC-1; also known as GDF-15). A subset of the members of this category would be sufficient to represent the category as such in a predictive model.

Suitable SNPs related to other processes than PCa further include, but are not limited to rs3817334, rs10767664, rs2241423, rs7359397, rs7190603, rs571312, rs29941, rs2287019, rs2815752, rs713586, rs2867125, rs9816226, rs10938397, and rs1558902 all being related to the BMI of an individual. Other suitable SNP related to BMI are disclosed in the report "Contribution of 32 GWAS-identified common variants to severe obesity in European adults referred for bariatric surgery " by Mägi and co-authors as published in PLoS One. 2013 Aug 7;8(8):e70735. It is possible to define a parameter category as "SNP related to expression level of BMI" which includes SNP related to the BMI of the individual. A subset of the members of this category would be sufficient to represent the category as such in a predictive model.

There is provided a method, wherein a defined amount of SNP(s) related to PCa used in said method are at least 50 SNPs, such as at least 55, 60, 65, 60, 75, 80, 85, 90, 95, 100, 105, 110, 115, 120, 130, 140, 150, 160, 170, 180, 190, 200, 210, 220, 230, 240, 250, 260, 270, 280, 290 or 300 SNP(s).

The method herein also allows disregarding a subset of data of about 10% and optionally up to about 30%, such as about 15%, 20% or 30%, of the SNP(s) when forming the composite value.

The SNP(s) used in a method herein may be selected from the SNPs of any one of lists 1, 2 or 3 presented herein, or as further mentioned herein in any context.

When SNPs is used in a method as defined herein in the context of PCa or aPCa, this list may be combined with one or more of the biomarkers selected from the group consisting of: PSA, free PSA, hK2, MIC-1 and MSMB, such as 1, 2, 3, 4, 5, or 6, from this group, such as at least 3 biomarkers.

When a list of SNPs is used in a method as defined herein in the context of PCa or aPCa, this list may be combined with one or more of the biomarkers selected from the group consisting of: PSA, free PSA, intact PSA, hK2, MIC-1 and MSMB, such as 1, 2, 3, 4, 5, or 6, from this group, such as at least 3 biomarkers.

The determination of the genetic status may be conducted by use of MALDI mass spectrometry, as previously mentioned herein.

There is also provided herein the use of an assay device for performing a method as defined herein, said assay device comprising a solid phase having immobilised thereon at least three different categories of ligands, wherein:

- the first category of said ligands binds specifically to a defined amount of PCa related biomarker(s) as defined herein, and includes a plurality of different ligands binding specifically to each of said PCa related biomarker(s), and
- the second category of said ligands binds specifically to a defined amount of SNP(s) related to PCa as defined herein, or a subset thereof, and includes a plurality of different ligands binding specifically to each of said SNPs, and
- the third category of said ligands binds specifically to said PCa Genetic Subpopulation (PCaGS) SNP(s).

The PCa Genetic Subpopulation (PCaGS) SNP(s) comprise SNPs rs16901979, rs7818556, rs12793759 and rs138213197, which ligands binding thereto are all present on said assay device. Hence, in said assay device, the third category that bind specifically to the SNPs are selected from the group consisting of: rs16901979, rs7818556, rs12793759, and rs138213197.

Accordingly, there is provided herein the use of an assay device for performing a method as defined herein, wherein a PCa related biomarker(s) are any of the PCa related biomarkers previously defined herein and an SNP(s) related to PCa binding to the second category of ligands are any of the SNP(s) previously defined herein and presented in the lists (lists 1-4), or a subset thereof.

There is also provided the use of an assay device for performing a method as defined herein, wherein said assay device is present in a test kit, said test kit further comprising one or more detection molecules for specifically detecting the PCa related biomarker(s), the SNP(s) related to PCa and/or the PCa Genetic Subpopulation (PCaGS) SNP(s) bound to said first, second and third category of ligands, respectively.

Typically, one or more of the method steps as described in the below, are provided by means of a computer program product when executed in a computer comprising a processor and memory.

Hence, there is also provided herein a computer program product directly loadable into the internal memory of a digital computer, wherein the computer program product comprises software code means for at least performing the steps relating to combining data from said individual regarding said presence or concentration of a defined amount of PCa related biomarker(s), and data from said individual regarding PCa related genetic status to form either a PCaGS composite value or a general PCa population composite value and the steps concerning correlating said PCaGS composite value or general PCa population composite value to the presence of PCa or aggressive PCa in said individual by comparing the PCaGS composite value or general PCa population composite value to a pre-determined cut-off value established with control samples of known PCaGS/non-presence of PCa and control samples of known general PCa population PCa/ non-presence of PCa, respectively, for indicating a presence or non-presence of prostate cancer or aggressive prostate cancer in an individual. Hence herein, there is provided a computer program product directly loadable into the internal memory of a digital computer, wherein said computer program comprises software code means for performing at the above mentioned steps.

The above mentioned steps of the method may also be described as being conducted with a computer programmed to form or calculate composite values from the data of the above mentioned steps, and thereafter the method is conducted with a computer programmed to correlate the composite values to the presence or non-presence of PCa or aggressive PCa in said individual.

Hence, additionally, there is also provided herein a non-transitory, tangible computer readable storage medium having executable instructions to conduct such calculations or form such composite values and/or to conduct the correlation step as described above.

There is also provided herein, an apparatus comprising an assay device as defined herein and a computer program product.

As has been discussed previously, the assessment of the performance of PCa screening efficiency is difficult. Although the ROC-AUC characteristics provide some insight regarding performance, additional methods are desirable. One alternative method for assessing performance of PCa screening is to calculate the percentage of positive biopsies at a given sensitivity level and compare the performance of screening using PSA alone with any novel method for screening. This however requires that the performance of PSA is accurately defined.

One example of an assessment performance of PSA screening has been disclosed by IM Thompson and co-authors in the report "Assessing prostate cancer risk: results from the Prostate Cancer Prevention Trial." as published in J Natl Cancer Inst. 2006 Apr 19;98(8):529-34. In this report, prostate biopsy data from men who participated in the Prostate Cancer Prevention Trial (PCPT) was used to determine the sensitivity of PSA. In total, 5519 men from the placebo group of the PCPT who underwent prostate biopsy, had at least one PSA measurement and a digital rectal examination (DRE) performed during the year before the biopsy, and had at least two PSA measurements performed during the 3 years before the prostate biopsy was included. This report discloses that when using a PSA value of 3 ng/mL as a cutoff about 41% of the high-grade cancers (i.e. cancers with Gleason score 7 or above) will be missed.

A second analysis using the same study population has been disclosed by IM Thompson and co-authors in "Operating characteristics of prostate-specific antigen in men with an initial PSA level of 3.0 ng/ml or lower" as published in JAMA. 2005 Jul 6;294(1):66-70. In this report, the authors present an estimate of the sensitivity and specificity of PSA for all prostate cancer, Gleason 7+ and Gleason 8+. When using 3,1 ng/mL as PSA cut off value for biopsy a sensitivity of 56,7% and a specificity of 82,3% for Gleason 7+ tumors was estimated. In this report the authors concluded that there is no cut point of PSA with simultaneous high sensitivity and high specificity for monitoring healthy men for prostate cancer, but rather a continuum of prostate cancer risk at all values of PSA. This illustrates the complication with PSA as a screening test while still acknowledging the connection of PSA with prostate cancer.

One inevitable consequence of the difficulties in obtaining accurate and comparable estimates of the predictive performance of any given diagnostic or prognostic model in the screening of PCa is that when calculating the relative improvement of a novel method as compared to using PSA alone, the calculated relative improvement will vary depending on many factors. One important factor that influences the calculated relative improvement is how the control group (i.e. known negatives) is obtained. Since it is unethical to conduct biopsies on subjects where there are no indications of PCa, the control group will be selected with bias. Thus, the relative improvement of a novel method will depend on how the control group was selected, and there are multiple fair known methods to select control groups. Any reported estimated improvement must therefore be seen in the light of such variance. To the best of our experience, we estimate that if the relative improvement of a novel method is reported to be 15% as compared to the PSA value alone using one fair known method for selecting the control group, said novel method would be at least 10% better than the PSA value alone using any other fair known method for selecting the control group.

To become used in a widespread manner in society, the performance of a screen must meet reasonable health economic advantages. A rough estimate is that a screening method performing about 15% better than PSA (i.e. avoiding 15% of the unnecessary biopsies) at the same sensitivity level, i.e. detecting the same number of prostate cancers in the population, would have a chance of being used in a widespread manner in the current cost level of public health systems. However, for defined subpopulations of individuals a novel screening method may have economic advantages also for smaller improvements as compared to the PSA value performance. It is noted that even though significant efforts have been put on finding a combined model for the estimation of PCa risk (as exemplified in several of the cited documents in this patent application), only one such combined method is currently introduced in a limited manner in Stockholm, Sweden. There is currently no regular use of such combined methods in Europe. Thus, previous known multiparametric methods generally do not meet the socioeconomic standards to be useful in modern health care. The method of the current invention has better performance than previously presented combined methods and meet the socioeconomic performance requirements to at all be considered by a health care system.

The combination of data can be any kind of algorithmic combination of results, such as a linear combination of data wherein the linear combination improves the diagnostic performance (for example as measured using ROC-AUC). Other possible methods for combining into a model capable of producing a diagnostic estimate include (but are not limited to) non-linear polynomials, support vector machines, neural network classifiers, discriminant analysis, random forest, gradient boosting, partial least squares, ridge regression, lasso, elastic nets, k-nearest neighbors. Furthermore, the book "The Elements of Statistical Learning: Data Mining, Inference, and Prediction, Second Edition" by T Hastie, R Tibshirani and J Friedman as published by Springer Series in Statistics, ISBN 978-0387848570 describes many suitable methods for combining data in order to predict or classify a particular outcome.

The algorithm which turns the data from the different categories into a single value being indicative of if the patient is likely to suffer from PCa or aPCa is preferably a non-linear function, wherein the dependency of different categories is employed for further increasing the diagnostic performance of the method. For example, one important dependency is the measured level of a selected biomarker combined with any associated genetic marker related to the expected expression level of said biomarker. In cases where an elevated concentration of the biomarker is found in a patient sample, and at the same time said patient is genetically predisposed of having lower levels of said biomarkers, the importance of the elevated biomarker level is increased. Likewise, if a biomarker level is clearly lower than normal in a patient being genetically predisposed to have high levels of said biomarkers, the contradictory finding increases the importance of the biomarker level interpretation. The algorithm used for predicting the risk for regular or aggressive PCa may benefit from using transformed variables, for example by using the log10(PSA) value. Transformation is particularly beneficial for variables with a distribution that is deviating clearly from the normal distribution. Possible variable transformations include, but are not limited to, logarithm, inverse, square, and square root. It is further common to center each variable to zero average and unit variance.

EP 3 577 237 B1

Examples of how combining of data can be performed as a part of a method as defined herein, are presented e.g. in WO2014079865 (by applicant), and is further described herein.

Although the combining of data can be performed in different ways, a typical procedure according to the present invention can be illustrated in the following non-limiting manner.

In a typical case, data regarding biomarkers belonging to a parameter category will be combined according to a predetermined equation to form a composite value which is related to the risk related to the parameter category as such. One non-limiting example is to calculate the average value of all available measurement values (data) for the members of a biomarker category, and use said average value as the composite value representing said biomarker category. This procedure may clearly be applied regardless of how many biomarker members belong to the category. If only data for one of the biomarkers included in a category is available, it can be used in itself to represent the biomarker category. For biomarkers, the measured value commonly used in the step of combination of data is the concentration of said biomarker found in the biological sample. For example, for the biomarkers PSA and HK2, this is most commonly the concentration of biomarker in a blood sample as expressed in units ng/mL.

The genetic score (i.e. the genetics composite value, or more specifically the SNP composite value) calculation is typically based on a predetermined odds ratio for each individual SNP included in a parameter category. For each SNP the odds ratio, i.e. the likelihood that an individual who carries a SNP (i.e. has the risk allele defined by the SNP) has the disease or condition under study, is determined in advance. Determination of the odds ratio for a SNP is usually done in large prospective studies involving thousands of subjects with known conditions or diseases.

The genetic score for an individual can, as a non-limiting example, be computed according to the following algorithm: For the individual at test, each SNP is processed in the following manner. For each SNP the individual may carry two SNP risk alleles (homozygous positive for said SNP), or one risk allele (heterozygous positive for said SNP) or zero risk alleles (homozygous negative for said SNP). The number of alleles for a SNP is multiplied with the natural logarithm of the odds ratio for said SNP to form a risk assessment value for that particular SNP. This means that an individual who is negative for a particular SNP (i.e. has zero SNP risk alleles) will have no risk contribution from said particular SNP. This procedure is repeated for all SNP for which measurement data is available. When all risk assessment values have been calculated, the average of the risk contribution for the SNP for which measurement data are available is calculated and is used as the genetic score for said individual, i.e. the genetics composite value with respect to a certain category of SNPs. This procedure may clearly be applied regardless of how many SNP members belong to the SNP category. This procedure may further be applied to a small subset of defined (often very high-risk or very low-risk) SNP to define if an individual is member of a particular high-risk or low-risk subgroup.

In models predicting the risk for developing PCa or aPCa, there is often one or more cut-off values defined. The choice of cut-off value depends on many factors, including but not limited to the risk of the disease as such and the risk associated with inaccurately diagnosing an individual as positive who has not the disease (false positive). In the general case, a predictive model is usually a monotonic function $Y = f(x1, x2, ... ,xN)$ where the estimated risk of having the disease is correlated with the increasing value of Y. This means that if the cut-off value is set at a low level, the test will produce a large number of false positive results, but will on the other hand detect most individuals that actually have the disease. If the cut-off level is set at a high value the opposite occurs where individuals having a Y value above the cut-off level will with very high probability have the disease, but a large number of individuals with disease will receive a negative test results (i.e. large number of false negative results). The choice of cut-off level depends on many factors, including the socio-economic outcome of balancing (a) missing individuals with the disease and (b) treating individuals without the disease.

One rationale for special handling of subpopulations is illustrated in a hypothetical drawing (Figure 1). Figure 1 show 24 simulated data points, of them 20 following a simple linear relationship 101 and four data points 102 that are deviating from the bigger group. Assume that this complete data set of 24 data points represent a population and that a linear relationship between X and Y is applied to the complete data set of 24 data points. This results in the dotted line 110. While being mathematically correct, the dotted line 110 is unable to accurately describe the majority of the data (portion 101) because the deviating properties of portion 102 heavily affect the mathematical model. Even though this hypothetical illustration in Figure 1 is exaggerated for the purpose of clarity, it does illustrate the mathematical background of special handling of subgroups or subpopulations. Within a large data set such as population based screening of genetic and protein biomarkers, obtained data will often be heterogeneous with a dominating subpopulation that essentially follows a simple relationship between measured entities and observed outcome (e.g. biomarker concentration and disease state to mention one example). In addition to the dominating subpopulation there will be smaller subpopulations that exhibit a clearly different response pattern. The ability to identify and exclude deviating subgroups or subpopulations, such as 102, will improve performance of predictive models for the dominating subgroup 101.

When applied in practice, it will occasionally happen that one or a few measurements fail due to for example unforeseen technical problems, human error, or any other unexpected and uncommon reason. In such cases the data set obtained for an individual will be incomplete. Typically, such an incomplete data set would be difficult or even impossible to evaluate. However, the current invention relies on measurements of a large number of features of which many are partially redundant. This means that also for individuals for which the data set is incomplete, it will in many cases be possible to produce a high-quality assessment according to the invention. This is particularly true within categories, where for example the kallikrein-like biomarkers are correlated and partially redundant.

Technically, it is therefore possible to apply an algorithmic two-step approach, wherein the kallikrein biomarker contribution is summarized into a kallikrein score (or kallikrein value). This kallikrein score is then in a second step being combined with other data (such as genetic score, age, and family history to mention a few non-limiting examples) to produce a diagnostic or prognostic statement on PCa. Similar two-step procedures can be implemented for other classes of markers, such as genetic markers related to BMI or protein biomarkers related to transforming growth factor beta superfamily (a large family of structurally related cell regulatory proteins that includes MIC-1), to mention two non-limiting examples.

Genetic risk scores are also insensitive to small losses of data due to for example unforeseen technical problems, human error, or any other unexpected and uncommon reason. This is not due to redundancy because the contribution of one SNP to the risk score is typically not correlated to any other SNP. In the case of SNP, the risk change due to each SNP is small, and only by using multiple SNP related to a condition in concert, the risk change for said condition becomes large enough for having an impact on the model performance. This means that the impact any single SNP on the total result is typically small, and the omission of a few SNP will typically not alter the overall genetic score risk assessment in any large manner. The typical data loss in the large scale genetic measurements is on the order of 1-2%, meaning that if a genetic score is composed of 100 different SNP, the typical genetic characterization of an individual would provide information about 98-99 of these SNP's. In current state of the art, some models have been shown to withstand a larger loss in data, such as 5-7% loss of information, or 7-15%, or even 15-30%, such as disclosed in WO 2014079865. Hence, the present method is also based on a redundantly designed combination of data, as defined elsewhere herein.

One preferred method for combining information from multiple sources has been described in the public report "Polygenic Risk Score Improves Prostate Cancer Risk Prediction: Results from the Stockholm-1 Cohort Study" by Markus Aly and co-authors as published in EUROPEAN UROLOGY 60 (2011) 21 - 28. Associations between each SNP and PCa at biopsy were assessed using a Cochran-Armitage trend test. Allelic odds ratios (OR) with 95% confidence intervals were computed using logistic regression models. For each patient, a genetic risk score was created by summing the number of risk alleles (0, 1, or 2) at each of the SNPs multiplied by the logarithm of that SNP's OR. Associations between PCa diagnosis and evaluated risk factors were explored in logistic regression analysis. The portion of the model related to non-genetic information included logarithmically transformed total PSA, the logarithmically transformed free-to-total PSA ratio, age at biopsy, and family history of PCa (yes or no). A repeated 10-fold cross-validation was used to estimate the predicted probabilities of PCa at biopsy. Ninety-five percent confidence intervals for the ROC-AUC values were constructed using a normal approximation. All reported p values are based on two-sided hypotheses.

In some cases it is not always possible to distinguish between prostate cancer in general and aggressive prostate cancer in a subpopulations due to the required cohort size to be capable of using aggressive prostate cancer as end-point in a statistically sound manner. There are however many rational reasons for distinguishing between prostate cancer in general and aggressive prostate cancer when possible. In most cases, prostate cancer is a slowly progressing disease. The fact that most men are diagnosed late in life means that a large fraction of the men diagnosed with prostate cancer die of other causes. Thus, the ability to estimate if an individual is at elevated risk for having prostate cancer and in particular aggressive prostate cancer, prior to biopsy, makes it possible for example to motivate the individual to change life-style. To stop smoking, to reach a BMI value below 30 and to exercise regularly (approximately 30 minutes 3-6 days of the week) are all factors that in general promotes survival in conditions of severe disease, including prostate cancer. Hence, if an individual is found having elevated risk for PCa or aPCa it is reason to suggest to said individual to stop smoking, try to reach BMI<30 and start exercising. Another important aspect is dietary issues. Through changing the diet, the PCa development may be reduced or delayed. There is evidence suggesting that reduced dietary intake can reduce the risk for onset of PCa as reported by Song and co-authors in the publication "Whole milk intake is associated with prostate cancer-specific mortality among U.S. male physicians." as published in J Nutr. 2013 Feb; 143(2): 189-96. Similar evidence exists for the positive effects of intake of green tea and intake of soy products.

Hence, if an individual is found having elevated risk for PCa or aPCa it is reason to suggest to said individual to decrease intake of dairy products and increase intake of green tea and soy based products.

The invention is now further exemplified by the experimental section, but is not intended to be limited thereto.

EXPERIMENTAL SECTION

**Example 1**

In a first example, the PCaGS_ex1 subgroup was defined as the following:
A PCaGS_ex1 member has one or both of the following:

Homozygote risk allele carrier of SNP with odds ratio from 1.2 to 2
Heterozygote risk allele carrier of SNP with odds ratio >2

The data set used in the present example comprised 4384 individuals from the STHLM3 study, and for each of the individuals the genotype of 254 different SNP (list 2 above), protein biomarker concentrations (of total PSA, free total PSA, free intact PSA, hK2, MSMB and MIC1), family history, age, prostate volume and digital rectal examination results were known. 308 individuals (7%) were members of the PCaGS subpopulation. Of these 308, 60 (19%) had Gleason 7+ cancer.

The cohort of 4384 did not include information about ethnic background, but was a randomly selected cohort of men with residential address in Stockholm aged 50-70 years at the time. Sweden is a multicultural society. In 2012 about 700 000 of the residents (of about 9 million in total) were born outside Europe, predominantly in Asia. The age profile of Swedish residents who were born outside Europe is clearly different from the native population, so that higher ages (i.e. those being suitable for prostate cancer testing) are more prevalent. This means that the population in the cohort has clear influence from a variety of ethnicities.

Among the 254 SNP that were measured, the following ones have odds ratios greater than 1.2:

rs16901979 has odds ratio 1.44
rs7818556 has odds ratio 1.33
rs12793759 has odds ratio 1.29
rs138213197 (HOXB13) has odds ratio 3.5

When comparing the biomarker properties of the PCaGS_ex1 subpopulation to the remaining cohort of 4076 individuals (of which 701 (17%) had Gleason Score 7+ cancer), the following differences were found:
The performance of PSA was clearly different in the PCaGS_ex1 group as compared to the remaining cohort: For the PCaGS group, PSA alone had an AUC of 0.76 (as compared to an AUC of 0.60 on the remaining cohort). In fact, 47% of the PCaGS_ex1 individuals who had PSA > 4 ng/mL also had Gleason Score 7+ cancer, whereas in the remaining cohort of men with PSA > 4 only 31% had Gleason Score 7+ cancer.

Another clear difference was MIC-1: In the PCaGS _ex1 subpopulation a lower than average value was associated with increased cancer risk, whereas in the remaining cohort, a higher value than average was associated with increased cancer risk.

The fact that biomarker responses change indicates that the PCaGS_ex1 subpopulation has differences in the underlying biology, tentatively caused by key mutations of the genome. This means that by handling the PCaGS_ex1 subpopulation in a different manner than the remaining population, better diagnostic performance will be obtained for the PCaGS_ex1 subpopulation. One simple but powerful method to improve diagnostic performance for the PCaGS _ex1 group is to use one PCaGS_ex1-specific PSA cutoff value for the PCaGS_ex1 group and the conventional PSA cutoff value for the individuals who are not members of the PCaGS_ex1 subpopulation.

An alternative method for defining a high genetic risk group, PCaGS_ex1b, member is to require members to qualify according to one or more of the following:

Homozygote risk allele carrier of SNP with odds ratio from 1.2 to 2
Heterozygote risk allele carrier of two different SNP, each SNP with odds ratio from 1.2 to 2
Heterozygote risk allele carrier of SNP with odds ratio >2.
722 individuals (17%) were members of the PCaGS_ex1b subpopulation. Of these 722, 149 (21%) had Gleason 7+ cancer.

When comparing the biomarker properties of the PCaGS_ex1b subpopulation to the general populations, the PSA value cutoff would benefit from being different. Some regions in the world apply PSA=4 ng/mL as a cutoff for follow-up diagnostic procedures. To achieve comparable performance for the PCaGS_ex1b subgroup as for the general population, the PSA cutoff for PCaGS_ex1b would need to be approximately 3.6 ng/mL based on a comparison of performance to detect prostate cancer defined as Gleason Score 6 or greater. Based on the patient material used for this example, 69 individuals (1.5% of the cohort) were subjects belonging to the PCaGS_ex1b, subgroup and having PSA value between 3.6 ng/mL and 4 ng/mL, and these 69 individuals would be missed for follow-up if they would be handled according to the general population method.

It should be noted that the cohort used for this analysis is slightly biased for low PSA values, meaning that the PSA cutoff for PCaGS _exlb is of approximate nature. Repetition on a different cohort would be desirable to confirm the proper value of the PSA cutoff for PCaGS_ex1b.

## Example 2

In a second example, the same data set as in Example 1 was used, and the PCaGS _ex2 subgroup was defined as an individual carrying at least one risk allele of rs13 8213197 (HOXB13). The distribution of individuals with a PSA value greater than 4.0 ng/mL is shown in Table 3:

Table 3

|  | Benign | Gleason Score 6 or greater | Gleason score 7 or greater |
|---|---|---|---|
| PCaGS_ex2 group | 7 | 26 | 16 |
| Non-PCaGS_ex2 group | 1151 | 830 | 439 |

This means that for individuals in the PCaGS _ex2 subgroup with PSA value greater than 4.0 ng/mL, the probability of having prostate cancer (Gleason score 6 or greater) is approximately 79% and the probability of having aggressive prostate cancer (gleason score 7 or greater) is approximately 48%. In comparison, individuals that are not members of the PCaGS_ex2 subgroup and that have a PSA value greater than 4.0 have a 42% probability of prostate cancer and a 22% probability of aggressive prostate cancer. In this particular case where the PCaGS _ex2 group exhibits a strongly elevated risk profile when PSA > 4, it might be justifiable to bypass invasive diagnostic procedures (such as prostate biopsy) and proceed to treatment immediately.

Some regions in the world apply PSA=3 ng/mL as a cutoff for follow-up diagnostic procedures. When comparing the PSA value properties of the PCaGS_ex2 subpopulation to the general populations using the cutoff value 3 ng/mL as reference point, the PSA cutoff for PCaGS _ex2 subpopulation would need to be approximately 1.6 ng/mL to achieve comparable performance (based on a comparison of performance to detect aggressive prostate cancer defined as Gleason Score 7 or greater).

It should be noted that the cohort used for this analysis is free from bias for PSA >1 ng/mL from the standpoint of rs138213197 analysis.

## Example 3

In a third example, the same data set as in Example 1 was used, and the PCaGS _ex3 subgroup was defined by three SNPs: rs7818556 (with odds ratio = 1.33), rs9911515 (with odds ratio = 0.8813), rs620861 (with odds ratio = 0.9359). These three SNPs were combined into a subset score variable according to the equation below and subsequently used for defining the PCaGS_ex3 subgroup:

$$\text{Subset score} = \text{sum}(\ \langle\text{number of risk alleles}\rangle * \log(\text{odds ratio})\ )$$

$$\text{PCaGS \_ex3} = \text{individuals with subset score} < -0.20$$

Where "sum" indicates that subset score is the sum of the product of number of risk alleles and the log(odds ratio) for the three SNPs. The Subset score value ranged from - 0.39 to +0.57. The performance of the blood protein biomarker hk2 varied with subset score value: For individuals who had a subset score < -0.2 (n=926), hk2 had an AUC value of 0.60, whereas for the remaining cohort (individuals with subset score >= -0.2; n=3433) hk2 had an AUC value of 0.59. AUC is a difficult-to-interpret value and even a seemingly small increase can be of clinical value. Under all circumstances, this example illustrates that it is beneficial to handle subgroups because one class of information (a genetic subset score in this particular case) can aid determine the performance of a different class of information (a blood protein biomarker in this particular case).

It should be noted that the cohort used for this analysis is slightly biased for low PSA values, meaning that the findings are of approximate nature. Repetition on a different cohort would be desirable to confirm values reported.

## Example 4

In a fourth example, the same data set as in Example 1 was used, and for each of the individuals the genotype of approximately 100 different SNP (rs10086908, rs 1009, rs10094059, rs10107982, rs1016343, rs10178804, rs10199796, rs10807843, rs10875943, rs10896437, rs10993994, rs11091768, rs11168936, rs11568818, rs11601037, rs11649743, rs11900952, rs12151618, rs12475433, rs12490248, rs12500426, rs12543663, rs12793759, rs12946864, rs12947919, rs13265330, rs138213197, rs1482679, rs16860513, rs16901841, rs16901922, rs16901979, rs16902094, rs17021918, rs17138478, rs17224342, rs17467139, rs1873555, rs1894292, rs1933488, rs1992833, rs2025645, rs2028900, rs2047408, rs2107131, rs2132276, rs2270785, rs2297434, rs2315654, rs2331780, rs2366711, rs2465796, rs2473057, rs2659051, rs2660753, rs2710647, rs2735839, rs2823118, rs3019779, rs306801, rs3096702, rs3120137, rs3745233, rs3765065, rs4245739, rs4699312, rs4871779, rs4925094, rs5918762, rs5934705, rs5935063, rs5945619, rs5978944, rs6062509, rs6090461, rs620861, rs6489721, rs651164, rs6545962, rs6569371, rs6579002, rs6625760, rs6794467, rs684232, rs6983267, rs7102758, rs7106762, rs7213769, rs747745, rs749264, rs758643, rs7679673, rs7752029, rs7818556, rs888507, rs902774, rs9297746, rs9297756, rs9359428, rs9364554, rs9911515), protein biomarker concentrations (of total PSA, free total PSA, free intact PSA, hK2, MSMB and MIC1), family history, age, prostate volume and digital rectal examination results were known. 308 individuals (7%) were members of the PCaGS subpopulation. Of these 308, 60 (19%) had Gleason 7+ cancer.

The purpose of the present example is to illustrate the level of redundancy encompassed by the multitudes of SNP.

The data set was evaluated for predictive performance (as measured using the AUC value) for (a) all SNP included; (b) 90% (randomly selected) of SNP included, (c) 80% (randomly selected) of SNP included, and (d) 70% (randomly selected) of SNP included. For each level of SNP inclusion (except 100% inclusion), the evaluation of predictive performance was repeated 9 times (with different randomly selected subsets of SNPs). The performance of the model comprising all SNP, AUC for detecting Gleason 6 and higher (regular and aggressive) Prostate cancers was 0.667 and AUC for detecting Gleason 7 and higher (aggressive only) Prostate cancers was 0.740. The smallest AUC value for detecting Gleason 6 or higher among all randomly reduced data sets was 0.664. The smallest AUC value for detecting Gleason 7 or higher among all randomly reduced data sets was 0.737. Hence, from an overall perspective, the loss of up to 30% of SNP does not adversely impact the ability to detect Gleason 6 and higher nor Gleason 7 and higher Prostate cancers.

## Example 5

In a fifth example, a raw data set collected at later time point in the same study (with the approximately the same mix of ethnic backgrounds as described in example 1) was used, this updated raw data set comprising more than 7000 individuals. This raw data set was reduced by excluding all individuals with a PSA value < 3 ng/mL, leaving 4035 individuals for analysis. Of these 66 were HOXB13 carriers (rs138213197). The overall risk for aggressive Prostate cancer (Gleason Score $\geq$ 7) was 0.3 for HOXB13 carriers, meaning that 20 of the 66 individuals had aggressive Prostate cancer Gleason Score $\geq$ 7.

Using the following model, where HOXB13 is not handled in any particular manner, was developed:

Equation 1:

$$\log(p/(1-p)) = -4.24551005 + 0.14843733 * mic1 - 0.38243626 * msmb + 12.55589194 * hk2 + 1.28151969 * intact\ psa + 0.19889028 * total\ psa - 1.32888043 * free\ psa - 3.83565211 * ratio + 0.05372347 * age + 0.11347054 * score + 0.36517819 * fh - 1.29112652 * prevBiop + 1.09543252 * dre - 0.02766189 * volume$$

Where mic1, msmb, hk2, intact psa, total psa, and free psa refers to the blood biomarker concentrations of the respective proteins; where ratio is free psa / total psa; where score is the genetic composite score reflecting the overall genetic risk; where fh refers to family history (1 if father/brother has been diagnosed for PCa, else 0); where dre is the resolute of a digital rectal examination (1 if positive, else 0); and where volume is the volume of the prostate.

Using this model, the overall risk for HOXB13 carriers is estimated to 0.24, which is an underestimation.

Next, a model where HOXB13 carriers are explicitly handled was developed. This means that the model includes a term that discriminates between the general population and the HOXB13 subgroup.

Equation 2:

$$\log(p/(1-p)) = -4.26698201 + 0.14991726*mic1 - 0.38454609*msmb + 12.39441889*hk2 + 1.29100995*intact\ psa + 0.19610494\ total\ psa - 1.31565142*free\ psa - 3.90198926*ratio + 0.05417630*age + 0.09326416*score + 0.36655549*fh - 1.28523130*prevBiop + 1.09301427*dre - 0.02746414*volume + 0.41023941*hoxb13$$

Where mic1, msmb, hk2, intact psa, total psa, and free psa refers to the blood biomarker concentrations of the respective proteins; where ratio is free psa / total psa; where score is the genetic composite score reflecting the overall genetic risk; where fh refers to family history (1 if father/brother has been diagnosed for PCa, else 0); where dre is the resolute of a digital rectal examination (1 if positive, else 0); where volume is the volume of the prostate; and where hoxb13 indicates if the individual is a hoxb13 risk allele carrier (1 if true, else 0). Hence, the final term of the equation (0.41023941*hoxb13) will adjust the risk level for the genetic subgroup of HOXB13 positive men.

Using this HOXB13 model that distinguishes the HOXB13 subgroup from the remaining population, the overall risk for HOXB13 carriers is estimated to 0.3, which is an accurate estimation of the risk.

The performance of the two models on HOXB13 negative individuals is nearly identical: In both cases the overall risk for aggressive Prostate cancer was accurately estimated to 0.15. The small size of the HOXB13 subgroup limits the possibility for model improvements on the remainder of the population.

**Example 6**

The cohort as described in Example 1 was subjected to a more extensive analysis where examples 2 was included and amended with other potential groups of high-risk SNP suitable to define PCaGS.

PCaGS_ex2 = as defined in example 2 = Individuals who are HOXB13 positive (n=146)
PCaGS_61 = Individuals for which a PCaGS defining risk score containing (rs16901979, rs7818556, rs12793759, rs138213197) has a value greater than 0.7 (n=248)
PCaGS_62 = Individuals for which a PCaGS defining risk score containing (rs16901979, rs7818556, rs12793759, rs138213197, rs16860513, rs7106762) has a value greater than 0.7 (n=222)
PCaGS_63 = Individuals for which a PCaGS defining risk score containing SNPs listed in Table 1 and Table 2 has a value greater than 0.45 (n=277)

Table 4 displays sensitivity (sens) and specificity (spec) for PSA alone indicating presence of Prostate Cancer (Gleason Score = 6 or higher), applied both on the complete cohort and for the four defined PCaGS. At the commonly used PSA cut-off 3 ng/mL, PSA as applied on the complete cohort has a sensitivity of about 75% and a specificity of about 24%. A model comprising multiple protein biomarkers, genetic score, and clinical information, such as equation 1 in example 5 above, has approximately twice the specificity at the same sensitivity (i.e. specificity of approximately 48%). The PCaGS_ex2 would, in order to match the sensitivity of the commonly applied PSA=3ng/mL cutoff, require a PSA cut-off of about 1.6 ng/mL (as previously discussed in Example 2). For the PCaGS_ex2 to match the specificity of the commonly applied PSA=3ng/mL cut-off, a PCaGS specific PSA cutoff of 1.2-1.3 ng/mL would be required. For the subpopulation PCaGS_61, PCaGS specific PSA cut-offs of about 2.0 ng/mL and about 1.4-1.5 ng/mL are required to match the sensitivity and specificity of the commonly applied PSA=3ng/mL cut-off for general populations. In the same manner, for the subpopulation PCaGS_62, the PCaGS specific PSA cut-offs of about 1.9 ng/mL and about 1.4 ng/mL matches the sensitivity and specificity of the commonly applied PSA=3ng/mL cut-off for general populations, and for the PCaGS_63 the specific PSA cut-offs of about 2.6 ng/mL and about 2.4-2.5 ng/mL.

A similar reasoning can be conducted using PSA=4ng/mL as a commonly applied PSA cut-off for a general population, leading to other PCaGS specific PSA cut-offs mimicking the sensitivity or the specificity of PSA=4ng/mL for the general population.

This also means that while as the generic population would benefit from a complex risk assessment equations such as the equation 1 in example 5 above, a specific subpopulation such as PCaGS_ex2 can be subjected to a substantially simpler risk equation, because the performance of PSA alone with a cutoff value between 1.6 and 1.8 ng/mL is similar to the complex model as applied for a general population. Hence, measurements and data collection of nearly all elements in equation 1 in example 5 above can as an alternative be omitted for the PCaGS_ex2 subpopulation without severe loss of diagnostic performance. Such a limitation on required data amounts reduces the cost (fewer measurements need to be conducted) and potentially shortens the time to report the result to the individual (no need to wait for a large number of results from different measurements being collected).

Table 4 (bold - corresponding to PSA 3 ng/mL, bold/italics corresponding to PSA 4 ng/mL)

| PSA cutoff ng/mL | Complete cohort | | PCaGC_ex2 | | PCaGS_61 | | PCaGS_62 | | PCaGS_63 | |
|---|---|---|---|---|---|---|---|---|---|---|
| | Sens | Spec | Sens | Spec | sens | Spec | Sens | Spec | sens | spec |
| 1 | 99.71 | 0.71 | 98.63 | 12.33 | 99.21 | 8.2 | 99.12 | 9.26 | 99.35 | 0.81 |
| 1.1 | 99.3 | 1.76 | 98.63 | 17.81 | 99.21 | 14.75 | 99.12 | 14.81 | 99.35 | 0.81 |
| 1.2 | 98.48 | 2.62 | 94.52 | 21.92 | 96.83 | 18.03 | 96.49 | 18.52 | 98.7 | 3.25 |
| 1.3 | 97.73 | 3.34 | 94.52 | **26.03** | 96.83 | 20.49 | 96.49 | **21.3** | 98.05 | 5.69 |
| 1.4 | 96.74 | 4.5 | 87.67 | 31.51 | 92.86 | **23.77** | 92.11 | 25 | 96.75 | 8.94 |
| 1.5 | 95.8 | 5.32 | 79.45 | 35.62 | 87.3 | 26.23 | 85.96 | 27.78 | 96.1 | 9.76 |
| 1.6 | 95.05 | 6.45 | **76.71** | 41.1 | 84.92 | 29.51 | 84.21 | 31.48 | 94.81 | 9.76 |
| 1.7 | 94.23 | 7.61 | 73.97 | 42.47 | 83.33 | 31.97 | 82.46 | 33.33 | 94.81 | 12.2 |
| 1.8 | 93.01 | 8.7 | 71.23 | 49.32 | 80.95 | 35.25 | 79.82 | 37.04 | 93.51 | 13.01 |
| 1.9 | 91.72 | 9.56 | 67.12 | 57.53 | 78.57 | 40.98 | 77.19 | 42.59 | 90.91 | 13.01 |
| 2 | 90.15 | 10.83 | 67.12 | *58.9* | **75.4** | 43.44 | 73.68 | 44.44 | 87.66 | 16.26 |
| 2.1 | 89.34 | 12.14 | 65.75 | 63.01 | 74.6 | 45.9 | 72.81 | 47.22 | 85.71 | 18.7 |
| 2.2 | 87.88 | 13.38 | 64.38 | 64.38 | 72.22 | 46.72 | 70.18 | 48.15 | 83.77 | **21.14** |
| 2.3 | 87.3 | 14.17 | 64.38 | 69.86 | 71.43 | 51.64 | 69.3 | 53.7 | 83.12 | 21.95 |
| 2.4 | 86.48 | 14.92 | 64.38 | 71.23 | 71.43 | 52.46 | 69.3 | 54.63 | 79.87 | 23.58 |
| 2.5 | 85.26 | 15.82 | 63.01 | 71.23 | 69.84 | 53.28 | 67.54 | 54.63 | 77.27 | 27.64 |
| 2.6 | 83.8 | 16.6 | 60.27 | 72.6 | 67.46 | 54.1 | 64.91 | 55.56 | 75.97 | 29.27 |
| 2.7 | 82.69 | 17.17 | 58.9 | 73.97 | 65.87 | 55.74 | 63.16 | 57.41 | 75.32 | 29.27 |
| 2.8 | 81.47 | 18.22 | 58.9 | 76.71 | 64.29 | 57.38 | 61.4 | *59.26* | 74.68 | 30.08 |
| 2.9 | 80.59 | 19.3 | 57.53 | 79.45 | 63.49 | 59.02 | 60.53 | 61.11 | 74.03 | 32.52 |
| 3 | 75.76 | **24.18** | 49.32 | 80.82 | 58.73 | **59.84** | 55.26 | 62.04 | 70.13 | 39.02 |
| 3.1 | 72.61 | 28.49 | *46.58* | 82.19 | 57.14 | 61.48 | 53.51 | 63.89 | 70.13 | 42.28 |
| 3.2 | 68.47 | 34 | 45.21 | 82.19 | 56.35 | 63.93 | 52.63 | 65.74 | 64.94 | 46.34 |
| 3.3 | 66.03 | 37.22 | 43.84 | 83.56 | 54.76 | 66.39 | 50.88 | 68.52 | 60.39 | 50.41 |
| 3.4 | 62.41 | 41.94 | 43.84 | 84.93 | 53.17 | 68.03 | 50 | 70.37 | 56.49 | 54.47 |
| 3.5 | 60.14 | 44.75 | 39.73 | 84.93 | 50 | 68.85 | *46.49* | 71.3 | 54.55 | 55.28 |
| 3.6 | 56.64 | 48.46 | 39.73 | 86.3 | 47.62 | 73.77 | 43.86 | 75.93 | 51.95 | *58.54* |
| 3.7 | 54.6 | 51.24 | 38.36 | 87.67 | 46.83 | 74.59 | 42.98 | 76.85 | 50.65 | 63.41 |
| 3.8 | 51.69 | 53.94 | 35.62 | 90.41 | 43.65 | 77.05 | 39.47 | 79.63 | *46.75* | 65.85 |
| 3.9 | 49.88 | 56.6 | 35.62 | 90.41 | 42.06 | 78.69 | 38.6 | 81.48 | 46.1 | 69.92 |
| 4 | *47.49* | *59.56* | 34.25 | 90.41 | 40.48 | 79.51 | 36.84 | 81.48 | 44.16 | 71.54 |
| 4.1 | 45.92 | 61.62 | 34.25 | 90.41 | 40.48 | 80.33 | 36.84 | 81.48 | 42.86 | 73.17 |
| 4.2 | 43.65 | 64.13 | 34.25 | 94.52 | 40.48 | 83.61 | 36.84 | 85.19 | 41.56 | 75.61 |
| 4.3 | 42.19 | 65.97 | 34.25 | 94.52 | 40.48 | 83.61 | 36.84 | 85.19 | 39.61 | 76.42 |
| 4.4 | 39.74 | 68.22 | 34.25 | 94.52 | 38.1 | 83.61 | 34.21 | 85.19 | 35.71 | 76.42 |
| 4.5 | 37.7 | 69.83 | 32.88 | 94.52 | 37.3 | 84.43 | 33.33 | 85.19 | 33.77 | 78.05 |

When conducting the same analysis for detection of aggressive PCa (defined as Gleason Score = 7 or greater), suitable PCaGS PSA cut-off values to preserve the performance of PSA = 3 ng/mL as applied on a general population are shown in Table 5. Also in this case, a complex risk assessment equations similar to the equation 1 in example 5 above would lead to twice the specificity compared to using PSA = 3 ng/mL on a general population. In other words, a complex risk assessment equations such as the equation 1 in example 5 applied on a general population has specificity approximately 50% at sensitivity 82%, while PSA = 3 ng/mL on a general population has specificity approximately 26% and sensitivity approximately 82%.

Table 5

| Subgroup | PSA subgroup cutoff to match performance of PSA = 3 ng/mL as used for general population for detecting aggressive prostate cancer | |
| | Same sensitivity ng/mL (about) | Same specificity ng/mL (about) |
|---|---|---|
| PCaGS_ex2 | 2.5-2.7 (2.6 ± 0.1) | 1.3-1.5 (1.4 ± 0.1) |
| PCaGS_61 | 2.8-3.0 (2.9 ± 0.1) | 1.4-1.6 (1.5 ± 0.1) |
| PCaGS_62 | 2.7-2.9 (2.8 ± 0.1) | 1.4-1.6 (1.5 ± 0.1) |
| PCaGS_63 | 2.8-3.0 (2.9 ± 0.1) | 2.3-2.5 (2.4 ± 0.1 ) |

When using aggressive PCa as end-point, the power of subgrouping is even clearer. When applying only a PSA cut-off 1.85 for PCaGS_ex2, the diagnostic performance outperforms complex risk assessment equations similar to the equation 1 in example 5 for that particular subpopulation.

It is hence not essential to apply a complex risk assessment equations similar to the equation 1 in example 5 for the PCaGS _ex2 subgroup, as long as a PSA value is available. The same is true for PCaGS_61 where a PSA cutoff value of 2.6 ng/mL outperforms the performance complex risk assessment equation similar to the equation 1 in example 5. The same is true for PCaGS_62 but with a PSA cut-off value of 2.4 ng/mL.

Below, we present PSA PCaGS cut-off values to match performance of PSA = 3 ng/mL and 4 ng/mL, respectively, used for a general population but for detecting aggressive PCa or PCa for the four PCaGS mentioned above.

Table 6

| Subgroup | PSA subgroup cutoff to match performance of PSA = 3 ng/mL as used for general population for detecting prostate cancer | |
| | Same sensitivity ng/mL (about) | Same specificity ng/mL |
|---|---|---|
| PCaGS_ ex2 | 1.5-1.7 (1.6 ± 0.1) | 1.1-1.3 (1.2 ± 0.1) |
| PCaGS_61 | 1.9-2.1 (1.8 ± 0.1) | 1.3-1.5 (1.4 ± 0.1) |
| PCaGS_62 | 1.8-2.0 (1.9 ± 0.1) | 1.3-1.5 (1.4 ± 0.1) |
| PCaGS_63 | 2.5-2.7 (2.6 ± 0.1) | 2.3-2.5 (2.4 ± 0.1) |

Table 7

| Subgroup | PSA subgroup cutoff to match performance of PSA = 4 ng/mL as used for general population for detecting prostate cancer | |
| | Same sensitivity ng/mL (about) | Same specificity ng/mL (about) |
|---|---|---|
| PCaGS_ex2 | 3.0-3.2 (3.1 ± 0.1) | 1.9-2.1 (2.0 ± 0.1) |
| PCaGS_61 | 3.5-3.7 (3.6 ± 0.1) | 2.9-3.1 (3.0 ± 0.1) |
| PCaGS_62 | 3.4-3.6 (3.5 ± 0.1) | 2.7-2.9 (2.8 ± 0.1) |
| PCaGS_63 | 3.7-3.9 (3.8 ± 0.1) | 3.5-3.7 (3.6 ± 0.1) |

This example illustrates that it is possible to define genetic subpopulations (PCaGS) that may benefit from completely different cut-off values of biomarkers. This example also illustrates that particular subpopulations do not require complete panels of biomarkers or SNP to make possible use of complex risk assessment equations similar to the equation 1 in example 5, which in turn means that the data necessary for using a complex risk assessment equation can potentially be omitted for particular subgroups without loss of diagnostic performance.

**Claims**

1.  A method for indicating a presence or non-presence of prostate cancer (PCa) in an individual, said method comprising the steps of:

    a) performing a genetic analysis of a biological sample obtained from said individual comprising determining a presence or non-presence of risk alleles of a group of Single Nucleotide Polymorphisms (SNPs) related to a PCa Genetic Subpopulation (PCaGS), wherein if at least one of said risk alleles is/are present in said sample, said individual is determined to belong to said PCaGS, and if said at least one risk allele of said group of SNPs is not present in said sample, said individual is determined not to belong to said PCaGS, wherein said group of SNPs consists of rs16901979, rs7818556, rs12793759 and rs138213197;
    b) if in step a) said individual is determined to belong to a PCaGS, then determine and characterize one or more additional PCa related parameter(s) in said PCaGS individual to indicate a presence or a non-presence of PCa in said PCaGS individual, wherein said one or more additional PCa related parameter(s) in said individual comprises measuring a total PSA value in said individual;
    c) if said individual in step a) is determined not to belong to a PCaGS, then

    i) determine a presence or concentration of a defined amount of PCa related biomarker(s) in said individual;
    ii) determine a PCa related genetic status by determining a presence or absence of a defined amount of one or more risk alleles of a SNP(s) related to PCa in said individual;
    iii) combine data from said individual regarding said presence or concentration of a defined amount of PCa related biomarker(s), and data from said individual regarding a PCa related genetic status to form a general PCa population composite value;
    iv) correlate said general PCa population composite value to the presence or non-presence of PCa in said individual by comparing the general PCa population composite value to a pre-determined cut-off value established with control samples of known general PCa population and control samples of non-presence of PCa,

    wherein said defined amount of one or more risk allele(s) of an SNP related to PCa comprises SNPs present in any one of the following lists:

    rs138213197, rs7818556, rs6983267, rs10993994, rs12793759, rs16901979, rs9911515, rs1016343, rs7106762, rs6579002, rs16860513, rs5945619, rs16902094, rs10896437, rs651164, rs7679673, rs13265330, rs2047408, rs10107982, rs620861, rs9297746, rs1992833, rs7213769, rs2710647, rs888507, rs17021918, rs12500426, rs2028900, rs7102758, rs16901922, rs6062509, rs2659051, rs12543663, rs4699312, rs11091768, rs3120137, rs6794467, rs10086908, rs2315654, rs12151618, rs747745, rs1009, rs2132276, rs2735839, rs11568818, rs684232, rs9364554, rs2660753, rs10807843, rs1933488, rs17467139, rs12947919, rs2331780, rs1894292, rs2107131, rs6545962, rs11649743, rs758643, rs2297434, rs902774, rs17224342, rs5918762, rs17138478, rs3019779, rs1873555, rs12946864, rs12475433, rs3765065, rs4871779, rs10875943, rs11601037, rs6489721, rs11168936, rs9297756, rs11900952, rs6569371, rs7752029, rs5934705, rs3745233, rs1482679, rs749264, rs6625760, rs5978944, rs2366711, rs5935063, rs10199796, rs2473057, rs4925094, rs3096702, rs12490248, rs4245739, rs10094059, rs306801, rs2823118, rs2025645, rs9359428, rs10178804, rs6090461, rs2270785 , rs16901841, and rs2465796 (list 1);
    and/or
    rs582598, rs439378, rs2207790, rs1046011, rs10458360, rs7525167, rs10489871, rs7529518, rs4245739, rs4512641, rs10178804, rs11900952, rs1873555, rs10191478, rs6755901, rs6545962, rs721048, rs2710647, rs12612891, rs2028900, rs1009, rs12233245, rs6760417, rs10496470, rs10199796, rs12475433, rs16860513, rs12151618, rs3765065, rs13017302, rs12988652, rs871688, rs749264, rs3771570, rs4346531, rs6770955, rs12637074, rs2660753, rs13319878, rs6437715, rs2162185, rs1515542, rs2270785, rs9830294, rs1439024, rs6762443, rs888507, rs6794467, rs12490248, rs1477886, rs4833103, rs3796547, rs17779822, rs2366711, rs16849146, rs1894292, rs12640320, rs3805284, rs12500426, rs4699312, rs17021918, rs7679673, rs2047408, rs2647262, rs12506850, rs7658048, rs2078277, rs12505546, rs13113975, rs4246742, rs2736098, rs401681, rs11134144, rs10060513, rs40485, rs2087724, rs1482679, rs16901841, rs1295683, rs2070874, rs7752029, rs2018334, rs9358913, rs1140809, rs409558, rs3096702, rs9267911, rs2025645, rs9359428, rs6569371, rs2813532, rs1933488, rs712242, rs6934898, rs9456490, rs651164, rs3120137, rs9364554, rs9457937, rs10486562, rs10807843, rs7801918, rs6962297, rs2465796, rs6957416, rs7777631, rs2272316, rs6961773, rs2132276, rs13265330, rs16887736, rs2911756, rs2272668, rs2339654, rs1380862, rs9297746, rs12543663, rs10086908, rs16901922, rs1016343, rs17832285, rs16901979, rs4871779, rs10107982, rs16902094, rs620861, rs17467139, rs6983267, rs9297756, rs10094059, rs7818556, rs1992833, rs986472, rs12552397, rs4273907, rs4237185, rs753032, rs11253002, rs2386841, rs10795841, rs10508422, rs7075945, rs10508678, rs539357, rs10826398, rs3818714, rs7090755, rs10993994, rs4382847, rs1891158, rs10887926, rs10788160, rs6579002, rs10832514, rs7358335, rs1944047, rs3019779, rs10896437, rs12793759, rs7106762, rs7102758, rs2449600, rs585197, rs2509867, rs11568818, rs7125415, rs11601037, rs11222496, rs4570588, rs6489721, rs3213764, rs17395631, rs4423250, rs11168936, rs10875943, rs3759129, rs902774, rs1827611, rs4760442, rs11610799, rs6539333, rs11067228, rs7485441, rs6489794, rs4119478, rs17070292, rs2293710, rs17256058, rs1950198, rs2331780, rs7141529, rs12880777, rs17123359, rs785437, rs524908, rs12903579, rs7178085, rs7164364, rs896615, rs11634741, rs9972541, rs12594014, rs11631109, rs1558902, rs8044335, rs2738571, rs885479, rs385894, rs684232, rs4925094, rs17138478, rs11649743, rs2107131, rs7213769, rs12946864, rs306801, rs138213197, rs1863610, rs17224342, rs9911515, rs12947919, rs966304, rs17744022, rs7234917, rs1943821, rs2227270, rs1363120, rs888663, rs1227732, rs1054564, rs4806120, rs11672691, rs758643, rs3745233, rs6509345, rs2659051, rs2735839, rs1354774, rs2691274, rs6090461, rs2297434, rs6062509, rs2315654, rs2823118, rs2838053, rs398146, rs16988279, rs2269640, rs4822763, rs132774, rs747745, rs5978944, rs6530238, rs5934705, rs5935063, rs4830488, rs17318620, rs5945619, rs5945637, rs11091768, rs2473057, rs5918762, rs4844228, rs6625760 and rs17324573 (list 2);
and/or
rs138213197, rs7818556, rs6983267, rs10993994, rs12793759, rs16901979, rs9911515, rs1016343, rs7106762, rs6579002, rs16860513, rs5945619, rs16902094, rs10896437, rs651164, rs7679673, rs13265330, rs2047408, rs10107982, rs620861, rs9297746, rs1992833, rs7213769, rs2710647, rs888507, rs17021918, rs12500426, rs2028900, rs7102758, rs16901922, rs6062509, rs2659051, rs17832285, rs12543663, rs4699312, rs11091768, rs3120137, rs6794467, rs10086908, rs7141529, rs2315654, rs12151618, rs747745, rs1009, rs2132276, rs2735839, rs11568818, rs684232, rs9364554, rs9830294, rs2660753, rs10807843, rs1933488, rs17467139, rs12947919, rs721048, rs385894, rs2331780, rs1894292, rs2107131, rs6545962, rs11649743, rs758643, rs2297434, rs902774, rs2647262, rs17224342, rs5918762, rs11672691, rs17138478, rs3019779, rs1873555, rs9457937, rs2838053, rs12946864, rs12475433, rs3765065, rs2018334, rs3771570, rs4871779, rs10875943, rs11601037, rs6489721, rs11168936, rs9297756, rs11900952, rs6569371, rs7752029, rs5934705, rs3745233, rs1482679, rs749264, rs6625760, rs5978944, rs2366711, rs5935063, rs10199796, rs2473057, rs4925094, and rs3096702 (list 3);
and/or
rs10086908, rs1009, rs10094059, rs10107982, rs1016343, rs10178804, rs10199796, rs10807843, rs10875943, rs10896437, rs10993994, rs11091768, rs11168936, rs11568818, rs11601037, rs11649743, rs11900952, rs12151618, rs12475433, rs12490248, rs12500426, rs12543663, rs12793759, rs12946864, rs12947919, rs13265330, rs138213197, rs1482679, rs16860513, rs16901841, rs16901922, rs16901979, rs16902094, rs17021918, rs17138478, rs17224342, rs17467139, rs1873555, rs1894292, rs1933488, rs1992833, rs2025645, rs2028900, rs2047408, rs2107131, rs2132276, rs2270785, rs2297434, rs2315654, rs2331780, rs2366711, rs2465796, rs2473057, rs2659051, rs2660753, rs2710647, rs2735839, rs2823118, rs3019779, rs306801, rs3096702, rs3120137, rs3745233, rs3765065, rs4245739, rs4699312, rs4871779, rs4925094, rs5918762, rs5934705, rs5935063, rs5945619, rs5978944, rs6062509, rs6090461, rs620861, rs6489721, rs651164, rs6545962, rs6569371, rs6579002, rs6625760, rs6794467, rs684232, rs6983267, rs7102758, rs7106762, rs7213769, rs747745, rs749264, rs758643, rs7679673, rs7752029, rs7818556, rs888507, rs902774, rs9297746, rs9297756, rs9359428, rs9364554, and rs9911515 (list 4),
or a subset of any one of lists 1-4, said subset comprising about 90%, 80%, 75% or 70% of the SNPs of any one of the lists 1-4;
and
wherein a defined amount of a PCa related biomarker(s) comprises one or more kallikrein-like PCa biomarker(s) and wherein at least one of the kallikrein-like PCa biomarkers is selected from the group consisting of (i) PSA, (ii) total PSA (tPSA), (iii) free PSA (fPSA), and (iv) hK2.

2. The method of claim 1, wherein said prostate cancer is an aggressive prostate cancer.

3. The method of claim 1 or 2, wherein the PCa related biomarker(s) further comprises MIC-1 and optionally other MIC-1 related biomarkers, and/or the biomarker MSMB and optionally other MSMB related biomarkers.

4. The method of any one of claims 1 to 3, wherein a total PSA cut-off value of step b) of claim 1 for indicating a presence of prostate cancer in a PCaGS individual is significantly lower than a standard general population total PSA cut-off value for indicating a presence of prostate cancer, such as at least about 10%, such as 10%, 20%, 30% 40% or even 50% lower than a standard cut-off value.

5. The method of claim 4, wherein the total PSA cut-off value of step b) of claim 1 for indicating a presence of prostate cancer in said PCaGS individual is about 1.8 to about 2.0 ng/ml or about 1.3 to about 1.5 ng/mL, to match a performance of total PSA of about 3 ng/mL, with regards to sensitivity and specificity, respectively, used for a general population for detecting prostate cancer.

6. The method of claim 1, wherein in step a) an individual is determined to belong to a PCaGS if said individual carries at least one risk allele of rs 13 8213197.

7. The method of claim 6, wherein a total PSA cut-off value of step b) of claim 1 for indicating a presence of prostate cancer in said PCaGS individual is significantly lower than a standard general population total PSA cut-off value for indicating a presence of prostate cancer, such as at least about 10%, such as 10%, 20%, 30% 40% or even 50% lower than a standard cut-off value.

8. The method of claim 7, wherein the total PSA cut-off value for indicating a presence of prostate cancer in said PCaGS individual is about 1.5 to about 1.7 ng/mL or about 1.1 to about 1.3 ng/mL to match a performance of total PSA of about 3 ng/mL, with regards to sensitivity and specificity, respectively, used for a general population for detecting prostate cancer.

9. The method of any one of the preceding claims, further comprising recommending the individual for biopsy if the composite value is greater than the cut-off value.

10. The method of any one of the preceding claims, further comprising collecting the family history regarding PCa, treatment history, and physical data from said individual; and wherein said family history, treatment history and/or physical data are included in the combined data forming said composite value.

11. Use of an assay device for performing a method according to any one of the preceding claims, said assay device comprising a solid phase having immobilised thereon at least three different categories of ligands, wherein:

   - the first category of said ligands binds specifically to said defined amount of PCa related biomarker(s), and includes a plurality of different ligands binding specifically to each of said PCa related biomarker(s), and
   - the second category of said ligands binds specifically to said defined amount of SNP(s) related to PCa, or a subset thereof, and includes a plurality of different ligands binding specifically to each of said SNPs, and
   - the third category of said ligands binds specifically to said PCa Genetic Subpopulation (PCaGS) SNP(s).

12. Use of of claim 11, wherein a PCa related biomarker(s) further comprises MIC-1 and optionally other MIC-1 related biomarkers, and/or the biomarker MSMB and optionally other MSMB related biomarkers.

13. Use of claim 11 or 12, wherein said assay device is present in a test kit, said test kit further comprising one or more detection molecules for specifically detecting the PCa related biomarker(s), the SNP(s) related to PCa and the PCa Genetic Subpopulation (PCaGS) SNP(s) bound to said first, second and third category of ligands, respectively.

14. A computer program comprising computer-executable instructions for causing a computer, when the computer-executable instructions are executed on a processing unit comprised in the computer, to perform at least steps iii) and iv) of step c) of claim 1.

**Patentansprüche**

1. Verfahren zum Anzeigen eines Vorhandenseins oder Nichtvorhandenseins von Prostatakrebs (PCa) in einem Individuum, das Verfahren umfassend die Schritte:

   a) Durchführen einer genetischen Analyse einer biologischen Probe, die von dem Individuum erhalten wird, umfassend das Bestimmen eines Vorhandenseins oder Nichtvorhandenseins von Risikoallelen einer Gruppe von Einzelnukleotidpolymorphismen (SNPs), die sich auf eine PCa-genetische Subpopulation (PCaGS) beziehen, wobei, wenn mindestens eines der Risikoallele in der Probe vorhanden ist/sind, bestimmt wird, dass das Individuum zu der PCaGS gehöret, und wenn das mindestens eine Risikoallel der Gruppe von SNPs nicht in der Probe vorhanden ist, bestimmt wird, dass das Individuum nicht zu der PCaGS gehört, wobei die Gruppe von SNPs aus rs16901979, rs7818556, rs12793759 und rs138213197 besteht;

b) wenn in Schritt a) bestimmt wird, dass das Individuum zu einer PCaGS gehört, dann Bestimmen und Charakterisieren eines oder mehrerer zusätzlicher PCa-bezogener Parameter in dem PCaGS-Individuum, um ein Vorhandensein oder ein Nichtvorhandensein von PCa in dem PCaGS-Individuum anzuzeigen, wobei der eine oder die mehreren zusätzlichen PCa-bezogenen Parameter in dem Individuum das Messen eines Gesamt-PSA-Werts bei den Individuum umfassen;

c) wenn bestimmt wird, dass das Individuum in Schritt a) nicht zu einer PCaGS gehört, dann

i) Bestimmen eines Vorhandenseins oder einer Konzentration einer definierten Menge an PCa-bezogenen Biomarkern in dem Individuum;

ii) Bestimmen eines PCa-bezogenen genetischen Status durch Bestimmen eines Vorhandenseins oder einer Abwesenheit einer definierten Menge eines oder mehrerer Risikoallele eines SNP(s) in Bezug auf PCa in dem Individuum;

iii) Kombinieren von Daten von dem Individuum hinsichtlich des Vorhandenseins oder der Konzentration einer definierten Menge von PCabezogenem/n Biomarker(n) und Daten von dem Individuum hinsichtlich eines PCa-bezogenen genetischen Status, um einen allgemeinen PCa-Populationsverbundwert zu bilden;

iv) Korrelieren des allgemeinen PCa-Populationsverbundwerts mit dem Vorhandensein oder dem Nichtvorhandensein von PCa in dem Individuum durch Vergleichen des allgemeinen PCa-Populationsverbundwerts mit einem vorbestimmten Grenzwert, der mit Kontrollproben der bekannten allgemeinen PCa-Population und Kontrollproben des Nichtvorhandenseins von PCa ermittelt wurde,

wobei die definierte Menge eines oder mehrerer Risikoallele eines SNP in Bezug auf PCa SNPs umfasst, die in einer der folgenden Listen vorhanden sind:

rs138213197, rs7818556, rs6983267, rs10993 994, rs12793759, rs16901979, rs9911515, rs1016343, rs7106762, rs6579002, rs16860513, rs5945619, rs16902094, rs10896437, rs651164, rs7679673, rs13265330, rs2047408, rs10107982, rs620861, rs9297746, rs1992833, rs7213769, rs2710647, rs888507, rs17021918, rs12500426, rs2028900, rs7102758, rs16901922, rs6062509, rs2659051, rs12543663, rs4699312, rs1I091768, rs3120137, rs6794467, rs10086908, rs2315654, rs12151618, rs747745, rs1009, rs2132276, rs2735839, rs11568818, rs684232, rs9364554, rs2660753, rs10807843, rs1933488, rs17467139, rs12947919, rs2331780, rs1894292, rs2107131, rs6545962, rs11649743, rs758643, rs2297434, rs902774, rs17224342, rs5918762, rs17138478, rs3019779, rs1873555, rs12946864, rs12475433, rs3765065, rs4871779, rs10875943, rs11601037, rs6489721, rs11168936, rs9297756, rs11900952, rs6569371, rs7752029, rs5934705, rs3745233, rs1482679, rs749264, rs6625760, rs5978944, rs2366711, rs5935063, rs10199796, rs2473057, rs4925094, rs3096702, rs12490248, rs4245739, rs10094059, rs306801, rs2823118, rs2025645, rs9359428, rs10178804, rs6090461, rs2270785, rs16901841 und rs2465796 (Liste 1);
und/oder
rs582598, rs439378, rs2207790, rs1046011, rs10458360, rs7525167, rs10489871, rs7529518, rs4245739, rs4512641, rs10178804, rs11900952, rs1873555, rs10191478, rs6755901, rs6545962, rs721048, rs2710647, rs12612891, rs2028900, rs1009, rs12233245, rs6760417, rs10496470, rs10199796, rs12475433, rs16860513, rs12151618, rs3765065, rs13017302, rs12988652, rs871688, rs749264, rs3771570, rs4346531, rs6770955, rs12637074, rs2660753, rs13319878, rs6437715, rs2162185, rs1515542, rs2270785, rs9830294, rs1439024, rs6762443, rs888507, rs6794467, rs12490248, rs1477886, rs4833103, rs3796547, rs17779822, rs2366711, rs16849146, rs1894292, rs12640320, rs3 805284, rs12500426, rs4699312, rs17021918, rs7679673, rs2047408, rs2647262, rs12506850, rs7658048, rs2078277, rs12505546, rs13113975, rs4246742, rs2736098, rs401681, rs11134144, rs10060513, rs40485, rs2087724, rs1482679, rs16901841, rs1295683, rs2070874, rs7752029, rs2018334, rs9358913, rs1140809, rs409558, rs3096702, rs9267911, rs2025645, rs9359428, rs6569371, rs2813532, rs1933488, rs712242, rs6934898, rs9456490, rs651164, rs3120137, rs9364554, rs9457937, rs10486562, rs10807843, rs7801918, rs6962297, rs2465796, rs6957416, rs7777631, rs2272316, rs6961773, rs2132276, rs13265330, rs16887736, rs2911756, rs2272668, rs2339654, rs1380862, rs9297746, rs12543663, rs10086908, rs16901922, rs1016343, rs17832285, rs16901979, rs4871779, rs10107982, rs16902094, rs620861, rs17467139, rs6983267, rs9297756, rs10094059, rs7818556, rs1992833, rs986472, rs12552397, rs4273907, rs4237185, rs753032, rs11253002, rs2386841, rs10795841, rs10508422, rs7075945, rs10508678, rs539357, rs10826398, rs3818714, rs7090755, rs10993 994, rs4382847, rs1891158, rs10887926, rs10788160, rs6579002, rs10832514, rs7358335, rs1944047, rs3019779, rs10896437, rs12793759, rs7106762, rs7102758, rs2449600, rs585197, rs2509867, rs11568818, rs7125415, rs11601037, rs11222496, rs4570588, rs6489721, rs3213764, rs17395631, rs4423250, rs11168936, rs10875943, rs3759129, rs902774, rs1827611, rs4760442, rs11610799, rs6539333, rs11067228, rs7485441, rs6489794, rs4119478, rs17070292, rs2293710, rs17256058, rs1950198, rs2331780, rs7141529, rs12880777, rs17123359, rs785437, rs524908, rs12903579, rs7178085, rs7164364, rs896615, rs11634741, rs9972541, rs12594014, rs11631109, rs1558902, rs8044335, rs2738571, rs885479, rs385894, rs684232, rs4925094, rs17138478, rs11649743, rs2107131, rs7213769, rs12946864, rs306801, rs138213197, rs1863610, rs17224342, rs9911515, rs12947919, rs966304, rs17744022, rs7234917, rs1943821, rs2227270, rs1363120, rs888663, rs1227732, rs1054564, rs4806120, rs11672691, rs758643, rs3745233, rs6509345, rs2659051, rs2735839, rs1354774, rs2691274, rs6090461, rs2297434, rs6062509, rs2315654, rs2823118, rs2838053, rs398146, rs16988279, rs2269640, rs4822763, rs132774, rs747745, rs5978944, rs6530238, rs5934705, rs5935063, rs4830488, rs17318620, rs5945619, rs5945637, rs11091768, rs2473057, rs5918762, rs4844228, rs6625760 und rs17324573 (Liste 2); und/oder

rs138213197, rs7818556, rs6983267, rs10993994, rs12793759, rs16901979, rs9911515, rs1016343, rs7106762, rs6579002, rs16860513, rs5945619, rs16902094, rs10896437, rs651164, rs7679673, rs13265330, rs2047408, rs10107982, rs620861, rs9297746, rs1992833, rs7213769, rs2710647, rs888507, rs17021918, rs12500426, rs2028900, rs7102758, rs16901922, rs6062509, rs2659051, rs17832285, rs12543663, rs4699312, rs11091768, rs3120137, rs6794467, rs10086908, rs7141529, rs2315654, rs12151618, rs747745, rs1009, rs2132276, rs2735839, rs11568818, rs684232, rs9364554, rs9830294, rs2660753, rs10807843, rs1933488, rs17467139, rs12947919, rs721048, rs385894, rs2331780, rs1894292, rs2107131, rs6545962, rs11649743, rs758643, rs2297434, rs902774, rs2647262, rs17224342, rs5918762, rs11672691, rs17138478, rs3019779, rs1873555, rs9457937, rs2838053, rs12946864, rs12475433, rs3765065, rs2018334, rs3771570, rs4871779, rs10875943, rs11601037, rs6489721, rs11168936, rs9297756, rs11900952, rs6569371, rs7752029, rs5934705, rs3745233, rs1482679, rs749264, rs6625760, rs5978944, rs2366711, rs5935063, rs10199796, rs2473057, rs4925094 und rs3096702 (Liste 3); und/oder

rs10086908, rs1009, rs10094059, rs10107982, rs1016343, rs10178804, rs10199796, rs10807843, rs10875943, rs10896437, rs10993994, rs11091768, rs11168936, rs11568818, rs11601037, rs11649743, rs11900952, rs12151618, rs12475433, rs12490248, rs12500426, rs12543663, rs12793759, rs12946864, rs12947919, rs13265330, rs138213197, rs1482679, rs16860513, rs16901841, rs16901922, rs16901979, rs16902094, rs17021918, rs1713 8478, rs17224342, rs17467139, rs1873555, rs1894292, rs1933488, rs1992833, rs2025645, rs2028900, rs2047408, rs2107131, rs2132276, rs2270785, rs2297434, rs2315654, rs2331780, rs2366711, rs2465796, rs2473057, rs2659051, rs2660753, rs2710647, rs2735839, rs2823118, rs3019779, rs306801, rs3096702, rs3120137, rs3745233, rs3765065, rs4245739, rs4699312, rs4871779, rs4925094, rs5918762, rs5934705, rs5935063, rs5945619, rs5978944, rs6062509, rs6090461, rs620861, rs6489721, rs651164, rs6545962, rs6569371, rs6579002, rs6625760, rs6794467, rs684232, rs6983267, rs7102758, rs7106762, rs7213769, rs747745, rs749264, rs758643, rs7679673, rs7752029, rs7818556, rs888507, rs902774, rs9297746, rs9297756, rs9359428, rs9364554 und rs9911515 (Liste 4), oder eine Teilmenge einer beliebigen der Listen 1 bis 4, die Teilmenge umfassend etwa 90 %, 80 %, 75 % oder 70 % der SNPs einer beliebigen der Listen 1 bis 4; und wobei eine definierte Menge eines PCa-bezogenen Biomarkers einen oder mehrere Kallikrein-ähnliche PCa-Biomarker umfasst und wobei mindestens einer der Kallikrein-ähnlichen PCa-Biomarker aus der Gruppe ausgewählt ist, bestehend aus (i) PSA, (ii) Gesamt-PSA (tPSA), (iii) freiem PSA (fPSA) und (iv) hK2.

2. Verfahren nach Anspruch 1, wobei der Prostatakrebs ein aggressiver Prostatakrebs ist.

3. Verfahren nach Anspruch 1 oder 2, wobei der/die PCa-bezogene/n Biomarker ferner MIC-1 und optional andere MIC-1-bezogene Biomarker und/oder den Biomarker MSMB und optional andere MSMB-bezogene Biomarker umfassen.

4. Verfahren nach einem der Ansprüche 1 bis 3, wobei ein Gesamt-PSA-Grenzwert von Schritt b) nach Anspruch 1 zum Anzeigen eines Vorhandenseins von Prostatakrebs in einem PCaGS-Individuum signifikant niedriger ist als ein standardmäßiger Gesamt-PSA-Grenzwert für die Allgemeinbevölkerung zum Anzeigen eines Vorhandenseins von Prostatakrebs, wie mindestens etwa 10 %, wie 10 %, 20 %, 30 % 40 % oder sogar 50 % niedriger als ein standardmäßiger Grenzwert.

5. Verfahren nach Anspruch 4, wobei der Gesamt-PSA-Grenzwert von Schritt b) nach Anspruch 1 zum Anzeigen eines Vorhandenseins von Prostatakrebs in dem PCaGS-Individuum etwa 1,8 bis etwa 2,0 ng/ml oder etwa 1,3 bis etwa 1,5 ng/ml beträgt, um hinsichtlich der Sensitivität beziehungsweise der Spezifität eine Leistung des Gesamt-PSA von etwa 3 ng/ml zu erreichen, die für die Erkennung von Prostatakrebs in der Allgemeinbevölkerung verwendet wird.

6. Verfahren nach Anspruch 1, wobei in Schritt a) bestimmt wird, dass ein Individuum zu einer PCaGS gehört, wenn das Individuum mindestens ein Risikoallel von rs138213197 trägt.

7. Verfahren nach Anspruch 6, wobei ein Gesamt-PSA-Grenzwert von Schritt b) nach Anspruch 1 zum Anzeigen eines Vorhandenseins von Prostatakrebs in einem PCaGS-Individuum signifikant niedriger ist als ein standardmäßiger Gesamt-PSA-Grenzwert für die Allgemeinbevölkerung zum Anzeigen eines Vorhandenseins von Prostatakrebs, wie mindestens etwa 10 %, etwa 10 %, 20 %, 30 % 40 % oder sogar 50 % niedriger als ein standardmäßiger Grenzwert.

8. Verfahren nach Anspruch 7, wobei der Gesamt-PSA-Grenzwert zum Anzeigen eines Vorhandenseins von Prostatakrebs in dem PCaGS-Individuum etwa 1,5 bis etwa 1,7 ng/ml oder etwa 1,1 bis etwa 1,3 ng/ml beträgt, um hinsichtlich der Sensitivität beziehungsweise der Spezifität eine Leistung des Gesamt-PSA von etwa 3 ng/ml zu erreichen, die für die Erkennung von Prostatakrebs in der Allgemeinbevölkerung verwendet wird.

9. Verfahren nach einem der vorstehenden Ansprüche, ferner umfassend ein Empfehlen des Individuums für Biopsie, wenn der Verbundwert größer als der Grenzwert ist.

10. Verfahren nach einem der vorstehenden Ansprüche, ferner umfassend ein Sammeln der Familiengeschichte hinsichtlich PCa, der Behandlungsgeschichte und physischen Daten des Individuums; und wobei die Familiengeschichte, die Behandlungsgeschichte und/oder physische Daten in den kombinierten Daten eingeschlossen sind, die den Verbundwert bilden.

11. Verwendung einer Testvorrichtung zum Durchführen eines Verfahrens nach einem der vorstehenden Ansprüche, die Testvorrichtung umfassend eine feste Phase, auf der mindestens drei verschiedene Kategorien von Liganden immobilisiert sind, wobei:

   - die erste Kategorie der Liganden spezifisch an die definierte Menge von PCa-bezogenem/n Biomarker(n) bindet, und eine Vielzahl von verschiedenen Liganden einschließt, die spezifisch an jeden der PCa-bezogenen Biomarker binden, und
   - die zweite Kategorie der Liganden spezifisch an die definierte Menge von SNP(s) bindet, die sich auf PCa beziehen, oder an eine Teilmenge davon, und eine Vielzahl von verschiedenen Liganden einschließt, die spezifisch an jeden der SNPs binden, und
   - die dritte Kategorie der Liganden spezifisch an die PCa-genetische Subpopulation (PCaGS) SNP(s) bindet.

12. Verwendung nach 11, wobei ein PCa-bezogener Biomarker ferner MIC-1 und optional andere MIC-1-bezogene Biomarker und/oder den Biomarker MSMB und optional andere MSMB-bezogene Biomarker umfasst.

13. Verwendung nach Anspruch 11 oder 12, wobei die Testorrichtung in einem Testkit vorhanden ist, das Testkit umfassend ferner ein oder mehrere Erkennungsmoleküle zum spezifischen Erkennen des/der PCa-bezogenen Biomarker(s), des/der SNP(s) in Bezug auf PCa und der PCa-Genetischen Subpopulation (PCaGS) SNP(s), die an die erste, die zweite beziehungsweise die dritte Kategorie von Liganden gebunden sind.

14. Computerprogramm, umfassend computerausführbare Anweisungen zum Veranlassen eines Computers mindestens die Schritte iii) und iv) von Schritt c) von Anspruch 1 auszuführen, wenn die computerausführbaren Anweisungen auf einer in dem Computer enthaltenen Verarbeitungseinheit ausgeführt werden.


**Revendications**

1. Procédé permettant d'indiquer une présence ou non-présence de cancer de la prostate (PCa) chez un sujet, ledit procédé comprenant les étapes consistant à :

   a) mettre en oeuvre une analyse génétique d'un échantillon biologique obtenu auprès dudit sujet comprenant la détermination d'une présence ou non-présence d'allèles à risque d'un groupe de polymorphismes mononucléotidiques (SNP) se rapportant à une sous-population génétique du PCa (PCaGS), dans lequel si au moins l'un desdits allèles à risque est/sont présent(s) dans ledit échantillon, ledit sujet est déterminé comme appartenant à ladite PCaGS, et si ledit au moins un allèle à risque dudit groupe de SNP n'est pas présent dans ledit échantillon, ledit sujet est déterminé comme n'appartenant pas à ladite PCaGS, dans lequel ledit groupe de SNP est constitué de rs16901979, rs7818556, rs12793759 et rs138213197 ;

b) si à l'étape a) ledit sujet est déterminé comme appartenant à une PCaGS, déterminer et caractériser alors un ou plusieurs paramètre(s) supplémentaire(s) se rapportant au PCa chez ledit sujet PCaGS pour indiquer une présence ou une non-présence de PCa chez ledit sujet PCaGS, dans lequel ledit ou lesdits paramètre(s) supplémentaire(s) se rapportant au PCa chez ledit sujet comprennent la mesurer une valeur totale de PSA chez ledit sujet ;

c) si ledit sujet à l'étape a) est déterminé comme n'appartenant pas à une PCaGS, alors

i) déterminer une présence ou une concentration d'une quantité définie de biomarqueur(s) se rapportant au PCa chez ledit sujet ;

ii) déterminer un statut génétique se rapportant au PCa en déterminant une présence ou absence d'une quantité définie d'un ou plusieurs allèles à risque d'un ou plusieurs SNP se rapportant au PCa chez ledit sujet ;

iii) combiner des données provenant dudit sujet concernant ladite présence ou concentration d'une quantité définie de biomarqueur(s) se rapportant au PCa, et des données provenant dudit sujet concernant un statut génétique se rapportant au PCa pour former une valeur composite de population PCa générale ;

iv) corréler ladite valeur composite de population PCa générale à la présence ou non-présence de PCa chez ledit sujet en comparant la valeur composite de population PCa générale à une valeur seuil prédéterminée établie avec des échantillons témoins d'une population PCa générale connue et des échantillons témoins de non-présence de PCa,

dans lequel ladite quantité définie d'un ou plusieurs allèle(s) à risque d'un SNP se rapportant au PCa comprend des SNP présents dans l'une quelconque des listes suivantes :

rs138213197, rs7818556, rs6983267, rs10993 994, rs12793759, rs16901979, rs9911515, rs1016343, rs7106762, rs6579002, rs16860513, rs5945619, rs16902094, rs10896437, rs651164, rs7679673, rs13265330, rs2047408, rs10107982, rs620861, rs9297746, rs1992833, rs7213769, rs2710647, rs888507, rs17021918, rs12500426, rs2028900, rs7102758, rs16901922, rs6062509, rs2659051, rs12543663, rs4699312, rs1I091768, rs3120137, rs6794467, rs10086908, rs2315654, rs12151618, rs747745, rs1009, rs2132276, rs2735839, rs11568818, rs684232, rs9364554, rs2660753, rs10807843, rs1933488, rs17467139, rs12947919, rs2331780, rs1894292, rs2107131, rs6545962, rs11649743, rs758643, rs2297434, rs902774, rs17224342, rs5918762, rs17138478, rs3019779, rs1873555, rs12946864, rs12475433, rs3765065, rs4871779, rs10875943, rs11601037, rs6489721, rs11168936, rs9297756, rs11900952, rs6569371, rs7752029, rs5934705, rs3745233, rs1482679, rs749264, rs6625760, rs5978944, rs2366711, rs5935063, rs10199796, rs2473057, rs4925094, rs3096702, rs12490248, rs4245739, rs10094059, rs306801, rs2823118, rs2025645, rs9359428, rs10178804, rs6090461, rs2270785, rs16901841 et rs2465796 (liste 1) ;
et/ou
rs582598, rs439378, rs2207790, rs1046011, rs10458360, rs7525167, rs10489871, rs7529518, rs4245739, rs4512641, rs10178804, rs11900952, rs1873555, rs10191478, rs6755901, rs6545962, rs721048, rs2710647, rs12612891, rs2028900, rs1009, rs12233245, rs6760417, rs10496470, rs10199796, rs12475433, rs16860513, rs12151618, rs3765065, rs13017302, rs12988652, rs871688, rs749264, rs3771570, rs4346531, rs6770955, rs12637074, rs2660753, rs13319878, rs6437715, rs2162185, rs1515542, rs2270785, rs9830294, rs1439024, rs6762443, rs888507, rs6794467, rs12490248, rs1477886, rs4833103, rs3796547, rs17779822, rs2366711, rs16849146, rs1894292, rs12640320, rs3805284, rs12500426, rs4699312, rs17021918, rs7679673, rs2047408, rs2647262, rs12506850, rs7658048, rs2078277, rs12505546, rs13113975, rs4246742, rs2736098, rs401681, rs11134144, rs10060513, rs40485, rs2087724, rs1482679, rs16901841, rs1295683, rs2070874, rs7752029, rs2018334, rs9358913, rs1140809, rs409558, rs3096702, rs9267911, rs2025645, rs9359428, rs6569371, rs2813532, rs1933488, rs712242, rs6934898, rs9456490, rs651164, rs3120137, rs9364554, rs9457937, rs10486562, rs10807843, rs7801918, rs6962297, rs2465796, rs6957416, rs7777631, rs2272316, rs6961773, rs2132276, rs13265330, rs16887736, rs2911756, rs2272668, rs2339654, rs1380862, rs9297746, rs12543663, rs10086908, rs16901922, rs1016343, rs17832285, rs16901979, rs4871779, rs10107982, rs16902094, rs620861, rs17467139, rs6983267, rs9297756, rs10094059, rs7818556, rs1992833, rs986472, rs12552397, rs4273907, rs4237185, rs753032, rs11253002, rs2386841, rs10795841, rs10508422, rs7075945, rs10508678, rs539357, rs10826398, rs3818714, rs7090755, rs10993 994, rs4382847, rs1891158, rs10887926, rs10788160, rs6579002, rs10832514, rs7358335, rs1944047, rs3019779, rs10896437, rs12793759, rs7106762, rs7102758, rs2449600, rs585197, rs2509867, rs11568818, rs7125415, rs11601037, rs11222496, rs4570588, rs6489721, rs3213764, rs17395631, rs4423250, rs11168936, rs10875943, rs3759129, rs902774, rs1827611, rs4760442, rs11610799, rs6539333, rs11067228, rs7485441, rs6489794, rs4119478, rs17070292,

EP 3 577 237 B1

rs2293710, rs17256058, rs1950198, rs2331780, rs7141529, rs12880777, rs17123359, rs785437, rs524908, rs12903579, rs7178085, rs7164364, rs896615, rs11634741, rs9972541, rs12594014, rs11631109, rs1558902, rs8044335, rs2738571, rs885479, rs385894, rs684232, rs4925094, rs17138478, rs11649743, rs2107131, rs7213769, rs12946864, rs306801, rs138213197, rs1863610, rs17224342, rs9911515, rs12947919, rs966304, rs17744022, rs7234917, rs1943821, rs2227270, rs1363120, rs888663, rs1227732, rs1054564, rs4806120, rs11672691, rs758643, rs3745233, rs6509345, rs2659051, rs2735839, rs1354774, rs2691274, rs6090461, rs2297434, rs6062509, rs2315654, rs2823118, rs2838053, rs398146, rs16988279, rs2269640, rs4822763, rs132774, rs747745, rs5978944, rs6530238, rs5934705, rs5935063, rs4830488, rs17318620, rs5945619, rs5945637, rs11091768, rs2473057, rs5918762, rs4844228, rs6625760 et rs17324573 (liste 2) ;
et/ou
rs138213197, rs7818556, rs6983267, rs10993994, rs12793759, rs16901979, rs9911515, rs1016343, rs7106762, rs6579002, rs16860513, rs5945619, rs16902094, rs10896437, rs651164, rs7679673, rs13265330, rs2047408, rs10107982, rs620861, rs9297746, rs1992833, rs7213769, rs2710647, rs888507, rs17021918, rs12500426, rs2028900, rs7102758, rs16901922, rs6062509, rs2659051, rs17832285, rs12543663, rs4699312, rs11091768, rs3120137, rs6794467, rs10086908, rs7141529, rs2315654, rs12151618, rs747745, rs1009, rs2132276, rs2735839, rs11568818, rs684232, rs9364554, rs9830294, rs2660753, rs10807843, rs1933488, rs17467139, rs12947919, rs721048, rs385894, rs2331780, rs1894292, rs2107131, rs6545962, rs11649743, rs758643, rs2297434, rs902774, rs2647262, rs17224342, rs5918762, rs11672691, rs17138478, rs3019779, rs1873555, rs9457937, rs2838053, rs12946864, rs12475433, rs3765065, rs2018334, rs3771570, rs4871779, rs10875943, rs11601037, rs6489721, rs11168936, rs9297756, rs11900952, rs6569371, rs7752029, rs5934705, rs3745233, rs1482679, rs749264, rs6625760, rs5978944, rs2366711, rs5935063, rs10199796, rs2473057, rs4925094 et rs3096702 (liste 3) ;
et/ou
rs10086908, rs1009, rs10094059, rs10107982, rs1016343, rs10178804, rs10199796, rs10807843, rs10875943, rs10896437, rs10993994, rs11091768, rs11168936, rs11568818, rs11601037, rs11649743, rs11900952, rs12151618, rs12475433, rs12490248, rs12500426, rs12543663, rs12793759, rs12946864, rs12947919, rs13265330, rs138213197, rs1482679, rs16860513, rs16901841, rs16901922, rs16901979, rs16902094, rs17021918, rs17138478, rs17224342, rs17467139, rs1873555, rs1894292, rs1933488, rs1992833, rs2025645, rs2028900, rs2047408, rs2107131, rs2132276, rs2270785, rs2297434, rs2315654, rs2331780, rs2366711, rs2465796, rs2473057, rs2659051, rs2660753, rs2710647, rs2735839, rs2823118, rs3019779, rs306801, rs3096702, rs3120137, rs3745233, rs3765065, rs4245739, rs4699312, rs4871779, rs4925094, rs5918762, rs5934705, rs5935063, rs5945619, rs5978944, rs6062509, rs6090461, rs620861, rs6489721, rs651164, rs6545962, rs6569371, rs6579002, rs6625760, rs6794467, rs684232, rs6983267, rs7102758, rs7106762, rs7213769, rs747745, rs749264, rs758643, rs7679673, rs7752029, rs7818556, rs888507, rs902774, rs9297746, rs9297756, rs9359428, rs9364554 et rs9911515 (liste 4),
ou un sous-ensemble de l'une quelconque des listes 1 à 4, ledit sous-ensemble comprenant environ 90 %, 80 %, 75 % ou 70 % des SNP de l'une quelconque des listes 1 à 4 ;
et
dans lequel une quantité définie d'un ou plusieurs biomarqueur(s) se rapportant au PCa comprend un ou plusieurs biomarqueur(s) de PCa de type kallikréine et dans lequel au moins l'un des biomarqueurs de PCa de type kallikréine est choisi dans le groupe constitué par (i) PSA, (ii) PSA total (tPSA), (iii) PSA libre (fPSA) et (iv) hK2.

2. Procédé selon la revendication 1, dans lequel ledit cancer de la prostate est un cancer de la prostate agressif.

3. Procédé selon la revendication 1 ou 2, dans lequel le(s) biomarqueur(s) se rapportant au PCa comprennent en outre MIC-1 et facultativement d'autres biomarqueurs se rapportant à MIC-1, et/ou le biomarqueur MSMB et facultativement d'autres biomarqueurs se rapportant à MSMB.

4. Procédé selon l'une quelconque des revendications 1 à 3, dans lequel une valeur seuil de PSA total de l'étape b) de la revendication 1 permettant d'indiquer une présence de cancer de la prostate chez un sujet PCaGS est significativement plus basse qu'une valeur seuil de PSA total de population générale standard pour indiquer une présence de cancer de la prostate, telle qu'au moins environ 10 %, telle que 10 %, 20 %, 30 % 40 % ou même 50 % plus basse qu'une valeur seuil standard.

5. Procédé selon la revendication 4, dans lequel la valeur seuil de PSA total de l'étape b) de la revendication 1 permettant d'indiquer une présence de cancer de la prostate chez ledit sujet PCaGS est d'environ 1,8 à environ 2,0 ng/mL ou d'environ 1,3 à environ 1,5 ng/mL, pour correspondre à une performance de PSA total d'environ 3 ng/mL, pour ce qui est de la sensibilité et de la spécificité, respectivement, utilisées pour une population générale pour détecter un cancer de la prostate.

6. Procédé selon la revendication 1, dans lequel à l'étape a) un sujet est déterminé comme appartenant à une PCaGS si ledit sujet porte au moins un allèle à risque de rs138213197.

7. Procédé selon la revendication 6, dans lequel une valeur seuil de PSA total de l'étape b) de la revendication 1 permettant d'indiquer une présence de cancer de la prostate chez ledit sujet PCaGS est significativement plus basse qu'une valeur seuil de PSA total de population générale standard pour indiquer une présence de cancer de la prostate, telle qu'au moins environ 10 %, telle que 10 %, 20 %, 30 % 40 % ou même 50 % plus basse qu'une valeur seuil standard.

8. Procédé selon la revendication 7, dans lequel la valeur seuil de PSA total permettant d'indiquer une présence de cancer de la prostate chez ledit sujet PCaGS est d'environ 1,5 à environ 1,7 ng/mL ou d'environ 1,1 à environ 1,3 ng/mL, pour correspondre à une performance de PSA total d'environ 3 ng/mL, pour ce qui est de la sensibilité et de la spécificité, respectivement, utilisées pour une population générale pour détecter un cancer de la prostate.

9. Procédé selon l'une quelconque des revendications précédentes, comprenant en outre le fait de recommander au sujet une biopsie si la valeur composite est supérieure à la valeur seuil.

10. Procédé selon l'une quelconque des revendications précédentes, comprenant en outre la collecte des antécédents familiaux concernant le PCa, des antécédents de traitement, et des données physiques auprès dudit sujet ; et dans lequel lesdits antécédents familiaux, antécédents de traitement et/ou données physiques sont inclus dans les données combinées formant ladite valeur composite.

11. Utilisation d'un dispositif de dosage pour mettre en oeuvre un procédé selon l'une quelconque des revendications précédentes, ledit dispositif de dosage comprenant une phase solide sur laquelle sont immobilisées au moins trois catégories différentes de ligands, dans lesquelles :

 - la première catégorie desdits ligands se lie spécifiquement à ladite quantité définie de biomarqueur(s) se rapportant au PCa, et comporte une pluralité de ligands différents se liant spécifiquement à chacun desdits biomarqueur(s) se rapportant au PCa, et
 - la deuxième catégorie desdits ligands se lie spécifiquement à ladite quantité définie du ou des SNP se rapportant au PCa, ou à un sous-ensemble de ceux-ci, et comporte une pluralité de ligands différents se liant spécifiquement à chacun desdits SNP, et
 - la troisième catégorie desdits ligands se lie spécifiquement audit ou auxdits SNP de sous-population génétique du PCa (PCaGS).

12. Utilisation selon la revendication 11, dans laquelle un ou des biomarqueur(s) se rapportant au PCa comprennent en outre MIC-1 et facultativement d'autres biomarqueurs se rapportant à MIC-1, et/ou le biomarqueur MSMB et facultativement d'autres biomarqueurs se rapportant à MSMB.

13. Utilisation selon la revendication 11 ou 12, dans laquelle ledit dispositif de dosage est présent dans une trousse de test, ladite trousse de test comprenant en outre une ou plusieurs molécules de détection permettant de détecter spécifiquement le(s) biomarqueur(s) se rapportant au PCa, le(s) SNP se rapportant au PCa et le(s) SNP de sous-population génétique du PCa (PCaGS) liés auxdites première, deuxième et troisième catégories de ligands, respectivement.

14. Programme d'ordinateur comprenant des instructions exécutables par ordinateur pour amener un ordinateur, lorsque les instructions exécutables par ordinateur sont exécutées sur une unité de traitement comprise dans l'ordinateur, à mettre en oeuvre au moins les étapes iii) et iv) de l'étape c) de la revendication 1.

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03100079 A
- US 2012021925 A
- WO 2012031207 A
- WO 2013172779 A
- WO 2014079865 A

### Non-patent literature cited in the description

- **MARKUS ALY.** Polygenic Risk Score Improves Prostate Cancer Risk Prediction: Results from the Stockholm-1 Cohort Study. *EUROPEAN UROLOGY,* 2011, vol. 60, 21-28
- **NICHOL MB.** Cost-effectiveness of Prostate Health Index for prostate cancer detection. *BJU Int.,* 11 November 2011
- **DAVID A. BROWN.** Macrophage Inhibitory Cytokine 1: A New Prognostic Marker in Prostate Cancer. *Clin Cancer Res,* 2009, vol. 15 (21), OF1-7
- **ROBERT KLEINS.** Blood Biomarker Levels to Aid Discovery of Cancer-Related Single-Nucleotide Polymorphisms: Kallikreins and Prostate Cancer. *Cancer Prev Res,* 2010, vol. 3 (5), 611-619
- **EWING CM.** Germline mutations in HOXB13 and prostate-cancer risk. *N Engl J Med.,* 12 January 2012, vol. 366 (2), 141-9
- **SHIIKI N.** Association between saliva PSA and serum PSA in conditions with prostate adenocarcinoma. *Biomarkers,* September 2011, vol. 16 (6), 498-503
- **MÄGI.** Contribution of 32 GWAS-identified common variants to severe obesity in European adults referred for bariatric surgery. *PLoS One.,* 07 August 2013, vol. 8 (8), e70735
- **IM THOMPSON.** Assessing prostate cancer risk: results from the Prostate Cancer Prevention Trial. *J Natl Cancer Inst.,* 19 April 2006, vol. 98 (8), 529-34
- **IM THOMPSON.** Operating characteristics of prostate-specific antigen in men with an initial PSA level of 3.0 ng/ml or lower. *JAMA.,* 06 July 2005, vol. 294 (1), 66-70
- The Elements of Statistical Learning: Data Mining, Inference, and Prediction. **T HASTIE ; R TIBSHIRANI ; J FRIEDMAN.** Springer Series in Statistics
- **MARKUS ALY.** Polygenic Risk Score Improves Prostate Cancer Risk Prediction: Results from the Stockholm-1 Cohort Study. *EUROPEAN UROLOGY,* 2011, vol. 60, 21-28
- Whole milk intake is associated with prostate cancer-specific mortality among U.S. male physicians. *J Nutr.,* February 2013, vol. 143 (2), 189-96